# EUROPEAN PATENT SPECIFICATION

(11) **EP 0 737 066 B1**
(45) Date of publication and mention of the grant of the patent: **02.04.2003**
(21) Application number: 95906742.2
(22) Date of filing: 09.01.1995
(51) Int. Cl.: A61K 9/70, A61K 47/32

(54) **TRANSDERMAL DEVICE CONTAINING POLYVINYLPYRROLIDONE AS SOLUBILITY ENHANCER**
ALS LÖSLICHKEIT-STEIGERNDE GEBRAUCHTE POLYVINYLPYRROLIDON ENTHALTENDE TRANSDERMALE VORRICHTUNG
DISPOSITIF D'ADMINISTRATION TRANSDERMIQUE CONTENANT DE LA POLYVINYLPYRROLIDONE EN TANT QU'AMPLIFICATEUR DE SOLUBILITE

(30) Priority: 07.01.1994 US 178558
(43) Date of publication of application: 16.10.1996
(73) Proprietor: NOVEN PHARMACEUTICALS, INC., Miami, FL 33186 (US)
(72) Inventor: MIRANDA, Jesus, Miami, FL 33186 (US); SABLOTSKY, Steven, Miami, FL 33175 (US)
(74) Representative: Werner, Hans-Karsten, Dr.Dipl.-Chem.
(86) International application number: PCT/US95/00022
(87) International publication number: WO 95/018603

(56) References cited:
- EP-A- 0 201 828
- EP-A- 0 272 045
- EP-A- 0 343 807
- EP-A- 0 416 842
- EP-A- 0 529 123
- WO-A-93/00058
- WO-A-93/08795

## Description

### Background of the Invention

This invention relates generally to transdermal drug delivery systems, and more particularly, to a transdermal drug delivery composition wherein a blend of polymers is utilized to affect drug solubility and the rate of drug delivery from the composition. More specifically, a plurality of polymers, preferably immiscible with each other, including a soluble polyvinylpyrrolidone ("PVP"), which can increase the maximum available concentration of the drug in the blend, thus permitting a major reduction in the size of the system needed to achieve therapeutic levels while maintaining desired delivery and adhesive properties.

The use of a transdermal composition, for example a pressure-sensitive adhesive containing a medicament, namely, a drug or other bioactive agent, as a means of controlling drug delivery through the skin at essentially a constant rate, is well known. Such known delivery systems involve incorporation of a medicament into a carrier such as a polymeric matrix and/or a pressure-sensitive adhesive composition. The pressure-sensitive adhesive must adhere effectively to the skin and permit migration of the medicament from the carrier through the skin and into the bloodstream of the patient.

Drug concentration in monolithic transdermal delivery systems can vary widely depending on the drug and polymers used. Low drug concentrations in the adhesive can result in difficulties in achieving an acceptable delivery rate of the medicament, preferably one approximating zero order kinetics. High drug concentrations, on the other hand, frequently affect the adhesion properties of the adhesives, and tend to promote crystallization. Crystallization occurs because, to varying degrees, most drugs which pass through the skin are not appreciably soluble or suspendible in pressure-sensitive adhesives.

In transdermal drug delivery systems, the presence of crystals (drugs or other additives or both) is generally undesirable. If the drug is present in crystalline form, it is not available for release from the system, and therefore not available for delivery. Moreover, although drug crystals can first dissolve and then release from the system, such a process is usually rate-limiting and tends to reduce transdermal permeation rates.

Simple diffusion models for permeation of drugs through the skin suggest that such permeation rates are concentration dependent, that is, dependent on both the amount and the degree of saturation of drug within the pressure-sensitive adhesive composition. Whereas polyacrylate adhesives have a high affinity for many drugs and thus tend to solubilize higher concentrations of drug than do rubber adhesives, difficulties in achieving acceptable permeation rates and adhesive properties are created when used as the only pressure-sensitive adhesive in a system. The minimal concentration at which the pressure-sensitive adhesive composition is saturated with drug in order to maximize permeation can be achieved by blending a rubber adhesive, which has little or no solubility for drugs, into a polyacrylate adhesive. However, the reduced solubilty and increased degree of supersaturation of drug in such a multiple polymer system results in a better environment for crystallization of the drug.

High concentrations of dissolved active ingredient can be used to increase flux of the active ingredient through the skin, as is shown in frequent reports of so called supersaturated systems.

Crystal size and distribution thus become important parameters which must be controlled in order to achieve maximum delivery of drug. These parameters are, however, usually difficult to control. Failure to control crystal size and distribution can result in products whose appearance suggests that the manufacturing process by which they are produced is not under control. More importantly, the presence of large crystals, particularly in excessive amounts, can be detrimental to adhesive-type transdermals. Crystals on the surface of the pressure-sensitive adhesive system can result in loss of adhesion. Furthermore, surface crystals can come into direct contact with the skin, and could cause skin irritation.

Soluble PVP is known as a crystallization inhibitor for transdermal preparations. However, PVP reduces or, in sufficiently high concentration, destroys acceptable permeation rates for delivery of a therapeutic level of drug and the adhesivity of pressure-sensitive adhesives. Schering AG EPO Patent Publication No. WO 93/08793, filed October 21, 1992, entitled "Transdermal Therapeutic Systems Containing Crystallization Inhibitors" describes PVP as a crystallization inhibitor in a single polymer adhesive system. Cygnus 5,252,334, granted October 12, 1993, entitled "Solid Matrix System for Transdermal Delivery" shows the use of PVP in a single polymer adhesive system without an enhancer.

Noven Pharmaceuticals, Inc. PCT US92/05297 filed June 22, 1992 and entitled "SOLUBILITY PARAMETER BASED DRUG DELIVER SYSTEM AND METHOD FOR ALTERING DRUG SATURATION CONCENTRATION" describes a monolithic transdermal delivery pressure-sensitive adhesive system comprising two different polymers. The rubber, having a lower solubility parameter, tends to decrease the solubility of the drug in the pressure-sensitive adhesive composition, thus lowering the concentration of solubilized drug.

None of the foreign patents and patent publications suggest that by adding a rubber to a drug in a polyacrylate adhesive, the rate of transdermal permeation can increase as a result of the increase in the degree of saturation, namely saturation or supersaturation, of the drug in the system. However, this increase in the degree of saturation can result in crystallization of the drug. Nor do these patents and patent publications suggest that the increased permeation rate need not be sacrificed in order to minimize the extent of crystallization or that the crystallization problem could be remedied by the addition of a soluble PVP, in an amount sufficient to solubilize all the drug in supersaturated concentration within the multiple polymer adhesive blend and yet maintain delivery of therapeutic levels of drug and also retain the adhesive properties of the composition.

### Summary of the Invention

It has now been found that soluble PVP can be used in a multiple polymer adhesive system in a narrow range to solubilize drugs in amounts similar to those which can be solubilized by a polyacrylate polymer system alone, without adversely affecting transdermal permeation rates, and permit the pressure-sensitive adhesive system blend to retain the needed adhesivity.

The foregoing and other objects are achieved by this invention by the inclusion of a soluble PVP in the blend of at least two polymers. The soluble PVP permits increased loading of the drug in the pressure-sensitive adhesive composition. The amount of soluble PVP used must be sufficient to solubilize the drug without undesirable crystallization and without substantially decreasing the permeation rate of drug or the adhesivity of the composition. The blend of at least two polymers is described in Noven Pharmaceuticals, Inc. PCT US92/05297 referred to above.

In accordance with one aspect of the invention, an improved pressure-sensitive adhesive composition comprises (1) a rubber, (2) a polyacrylate, (3) a drug and (4) a soluble PVP as defined in claim 1.

The term "supersaturated" used in reference to the drug means that the amount of drug present is in excess of its solubility or dispersability in a multiple polymer adhesive system which lacks the soluble PVP.

The term "polyvinylpyrrolidone," or "PVP" refers to a polymer, either a homopolymer or copolymer, containing N-vinylpyrrolidone as one of the monomeric units. Typical PVP polymers are homopolymeric PVPs and copolymers of vinyl acetate and vinylpyrrolidone. The homopolymeric PVPs are known to the pharmaceutical industry under a variety of designations including the generic name poly(1-vinyl-2-pyrrolidone) and the trademarks Povidone, Polyvidone, Polyvidonum and Polyvidonum. The copolymer vinyl acetate/vinylpyrrolidone is known to the pharmaceutical industry as Copolyvidon, Copolyvidone, and Copolyvidonum. Suitable PVP polymers include those sold under the trademark Kollidon by BASF AG, Ludwigshafen, Germany. Preferred are Kollidon 17PF, 25, 30, 90 and VA 64.

The term "soluble" when used with reference to PVP means that the polymer is soluble in water and generally is not substantially cross-linked, and has a molecular weight of less than about 2,000,000. See, generally, Bühler, KOLLIDON®: POLYVINYLPYRROLIDONE FOR THE PHARMACEUTICAL INDUSTRY, BASF AG (1992).

Although PVP can increase the solubility or dispersability of the drug within the multiple polymer adhesive system, increasing amounts of PVP leads to decreases in flux of the drug and decrease in adhesiveness of the system. Thus, the amount of soluble PVP selected should be sufficient to solubilize all the drug but insufficient to substantially retard flux of the drug from the system. This amount of drug can be experimentally determined but is generally in a drug to PVP ratio by weight of about 1:10 to about 10:1, preferably about 1:5 to about 5:1 and optimally about 1:3 to about 3:1.

Particularly preferred embodiments include blends comprising a rubber and a soluble PVP, wherein the rubber is a polysiloxane.

Polysiloxane is preferably present in the pressure-sensitive adhesive composition in an amount ranging from about 9% to about 94% by weight of the pressure-sensitive adhesive composition, while the polyacrylate is preferably present in an amount ranging up to about 85%. Preferably, the ratio of the polyacrylate to the rubber is from about 2:98 to about 96:4, and more preferably from about 2:98 to about 86:14 by weight. The optimum ratio of rubber to polyacrylate is that which permits the highest concentration of drug needed to achieve approximately zero order kinetics, and having sufficient soluble PVP to solubilize all the drug, without deleteriously affecting the adhesive properties of the pressure-sensitive adhesive composition or permeation rate of drug from the composition.

Soluble PVP is preferably present in the pressure-sensitive adhesive composition in an amount ranging from about 1% to about 20% by weight of the total composition in the ratio by weight to the drug as explained above. The minimum amount of soluble PVP to be added is that amount needed to increase the solubility of the drug in the ternary system to the solubility of the drug in an identical binary system, but which lacks the rubber. This amount can be determined experimentally by dissolving the desired amount of drug in the polyacrylate, then adding the desired amount of rubber, then adding sufficient soluble PVP to solubilize the drug. The actual amount to be used will depend on the system and can be experimentally determined by the addition of sufficient soluble PVP to the mixture to at least compensate for the reduction in solubility of the drug resulting from the addition of the rubber to the polyacrylate.

The maximum amount of soluble PVP to be added is that amount that permits delivery of a therapeutic amount of drug from the system, preferably with approximately zero order kinetics, and does not significantly reduce the adhesivity of the system needed for transdermal application. This amount can also be determined experimentally by measurement of flux or transdermal permeation rates from the system and measurement of adhesive properties of the system.

The pressure-sensitive adhesive composition of the invention comprises a blend of preferably about 9% to about 94% and optimally about 14% to about 94% by weight of a rubber, about 5% to about 85% by weight of a polyacrylate, and preferably about 1% to about 20%, more preferably about 3% to about 15% and optimally about 5% to about 15% by weight of a soluble PVP.

The multiple polymer adhesive system comprises about 50% to about 99% by weight of the pressure-sensitive adhesive composition. The multiple polymer adhesive system is combined with a drug in an amount of about 0.1% to 50%, optimally about 0.3% to about 30% by weight of the pressure-sensitive adhesive composition. Optional additives, such as co-solvents for the drug (up to 30% by weight) and enhancers (up to 20% by weight) may be included in the total composition.

In particularly preferred embodiments, the drug is a steroid, such as an estrogen or a progestational agent, or combination thereof. In other preferred embodiments, the drug may be a β₂-adrenergic agonist, such as albuterol, or a cardioactive agent, such as nitroglycerin. In still other embodiments, the drug is a cholinergic agent, such as pilocarpine, an antipsychotic such as haloperidol, a tranquilizer/sedative such as alprazolam, or an anesthetic or analgesic agents. Also, it has been recently recognized that CNS affecting drugs such as nicotine and selegiline can be administered transdermally within the scope of this invention.

The pressure-sensitive adhesive compositions may further include enhancers, fillers, co-solvents, and excipients as are known in the art for use in transdermal drug delivery compositions.

### Brief Description of the Drawings

Comprehension of the invention is facilitated by reading the following detailed description, in conjunction with the annexed drawings, in which:
FIG. 1 is a schematic illustration of a monolithic transdermal drug delivery device of the present invention;
FIG. 2 is a plot of diffusion coefficient versus net solubility parameter;
FIG. 3 shows the average flux of estradiol for two compositions of this invention containing a soluble PVP;
FIG. 4 shows estradiol flux through the human epidermis from PVP compositions of this invention;
FIG. 5 shows norethindrone acetate flux through human epidermis in a composition of this invention containing norethindrone acetate, estradiol and soluble PVP;
FIG. 6 shows average estradiol and norethindrone acetate flux from a composition of this invention containing varying concentrations of soluble PVP;

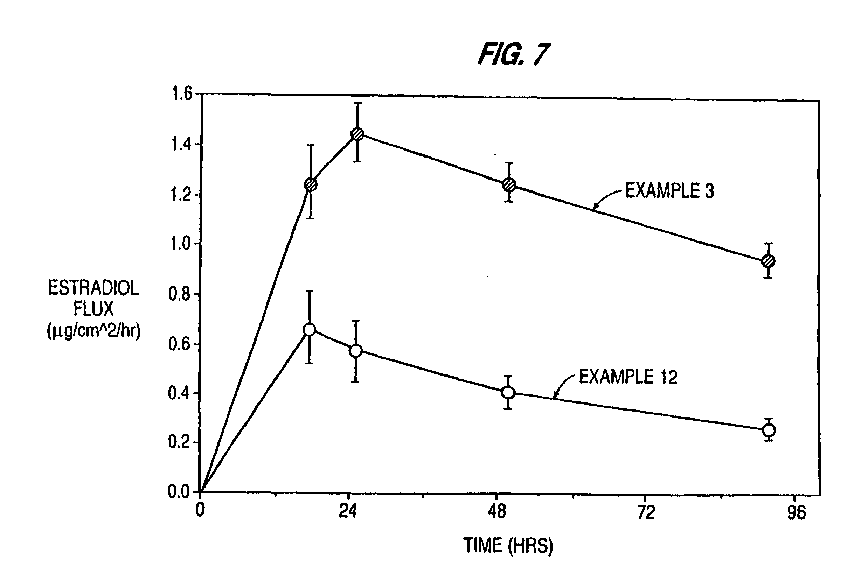

FIG. 7 shows effect of soluble PVP on estradiol flux through human epidermis;

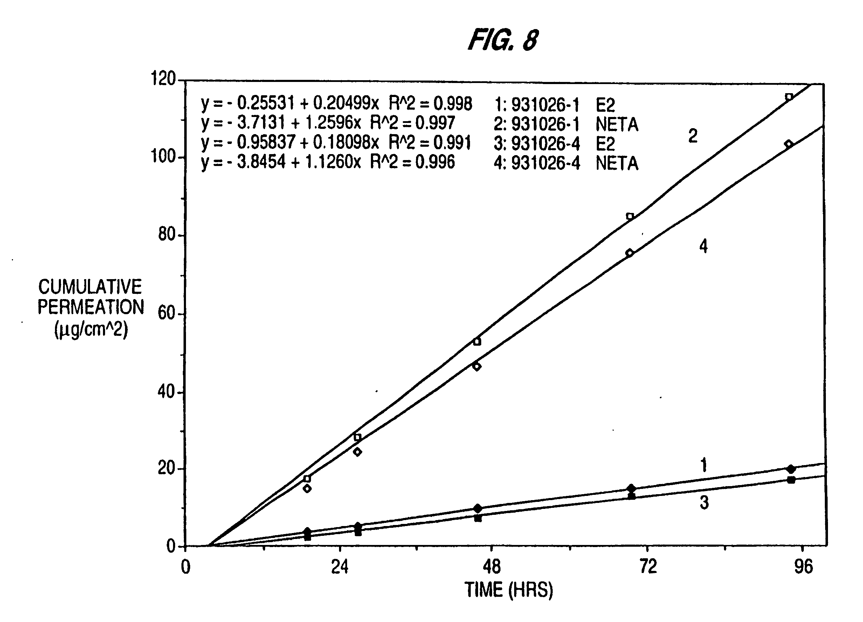

FIG. 8 shows cumulative permeation of estradiol and norethindrone acetate from a composition of this invention containing varying concentrations of soluble PVP;

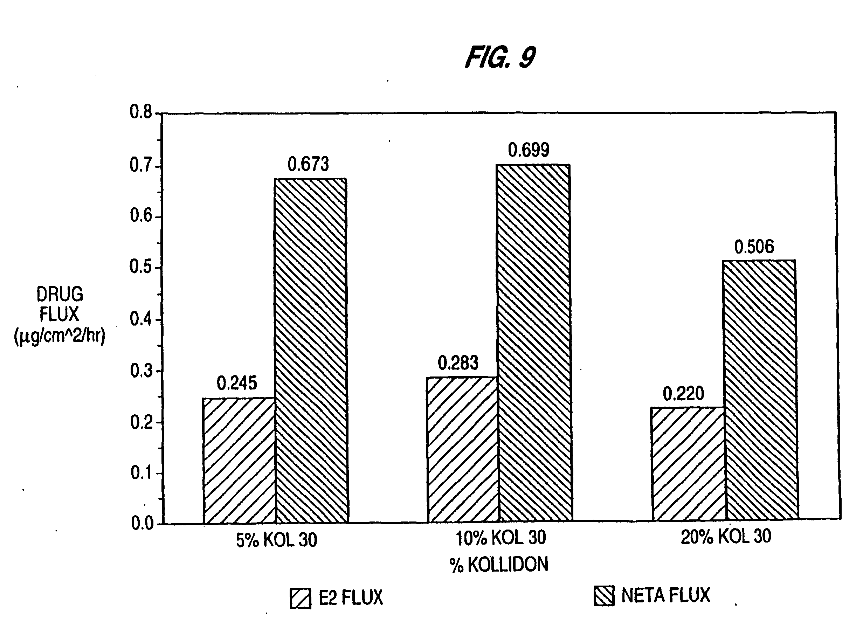

FIG. 9 shows effect of soluble PVP concentration on estradiol and norethindrone acetate flux through human epidermis from a composition of this invention; and

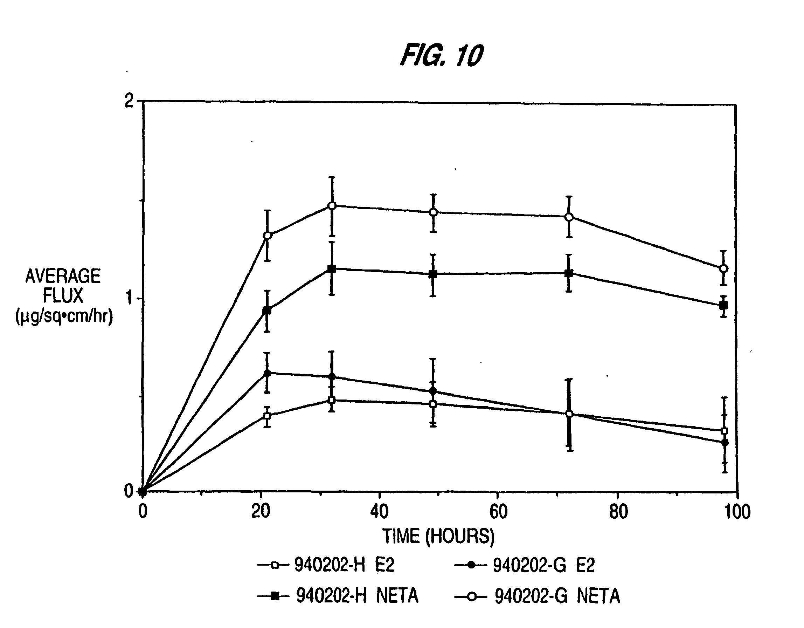

FIG. 10 shows effect of soluble PVP on average estradiol and norethindrone acetate flux from a composition of this invention containing varying concentrations of soluble PVP.

### Detailed Description of Preferred Embodiments

The invention relates to a pressure-sensitive adhesive composition comprising a blend of at least two polymers, a soluble PVP, and a drug. The blend of at least two polymers is herein referred to as a multiple polymer adhesive system. The term "blend" is used herein to mean that there is no, or substantially no, chemical reaction or cross-linking (other than simple H-bonding) between the different polymers in the multiple polymer adhesive system.

As used herein, the term "pressure-sensitive adhesive" refers to a viscoelastic material which adheres instantaneously to most substrates with the application of very slight pressure and remains permanently tacky. A polymer is a pressure-sensitive adhesive within the meaning of the term as used herein if it has the properties of a pressure-sensitive adhesive *per se* or functions as a pressure-sensitive adhesive by admixture with tackifiers, plasticizers or other additives. The term pressure-sensitive adhesive also includes mixtures of different polymers and mixtures of polymers, such as polyisobutylenes (PIB) of different molecular weights, the resultant mixtures being a pressure-sensitive adhesive. In the last case, the polymers of lower molecular weight in the mixture are not considered to be "tackifiers," said term being reserved for additives which differ other than in molecular weight from the polymers to which they are added.

As used herein, the term "rubber" refers to a viscoelastic material which has the properties of a pressure-sensitive adhesive and which contains at least one natural or synthetic elastomeric polymer. Suitable rubbers include polysiloxane, polyisobutylene and natural rubber.

As used herein, the term "drug," and its equivalents, "bioactive agent," and "medicament" are intended to have the broadest as including any therapeutically, prophylactically and/or pharmacologically or physiologically beneficial active substance, or mixture thereof, which is delivered to a living organism to produce a desired, usually beneficial, effect.

More specifically, any drug which is capable of producing a pharmacological response, localized or systemic, irrespective of whether therapeutic, diagnostic, or prophylactic in nature, in plants or animals is within the contemplation of the invention. Also within the invention are such bioactive agents as pesticides, insect repellents, sun screens, cosmetic agents, etc. It should be noted that the drugs and/or bioactive agents may be used singularly or as a mixture of two or more such agents, and in amounts sufficient to prevent, cure, diagnose or treat a disease or other condition, as the case may be.

The drug is used in a "pharmacologically effective amount." The latter term means that the concentration of the drug is such that in the composition it results. in a therapeutic level of drug delivered over the term that the transdermal dosage form is to be used, preferably with zero order kinetics. Such delivery is dependent on a great number of variables including the drug, the time period for which the individual dosage unit is to be used, the flux rate of the drug from the system and a number of other variables. The amount of drug needed can be experimentally determined based on the flux rate of the drug through the system and through the skin when used with and without enhancers. Having determined the flux rate needed, the transdermal delivery system is designed so that the release rate over the period of time of therapeutic use will be at least equal to the flux rate. Of course, the surface area of the transdermal delivery system also affects the delivery of the drug from the system.

The drug is present in a "supersaturated" amount as defined herein. The supersaturated amount is needed to increase the concentration of solubilized drug in the system. The soluble PVP is thus necessary to solubilize the drug in the otherwise supersaturated system.

In general, therapeutic amounts of drug can be delivered from the composition containing about 0.1% to about 50% by weight of drug. However, the composition of this invention is particularly useful for drugs which are used in relatively low concentrations, especially 0.3% to 30% of the total composition.

The soluble PVP is used in an amount effective to solubilize the drug, said amount being greater than that needed to solubilize the drug in an identical composition lacking a rubber. However, the maximum amount of soluble PVP used should be no greater than that which can maintain delivery of a therapeutic level of drug, preferably one which achieves zero order kinetics, and which can retain the adhesive properties of the pressure-sensitive adhesive composition for transdermal use. As an example, a steroid such as 17β-estradiol or norethindrone acetate, is soluble in the typical polyacrylate pressure-sensitive adhesive in an amount of approximately 2 to 4%, without the presence of added PVP, although, as noted in Schering AG PCT Patent Publication No. WO 93/08793 filed October 12, 1992, crystallization tends to occur in the absence of PVP and accelerates upon standing at room temperatures and pressures. The present inventors have found that the addition of a rubber to the mixture decreases the solubility of the steroid in the polyacrylate proportionally to the amount of rubber added. Thus, in a system containing an polyacrylate and a rubber, sufficient PVP must be used as a solubilizing agent to compensate for the lack of solubility created by the addition of the rubber. Moreover, only the minimum amount of soluble PVP should be added to the pressure-sensitive adhesive composition since increasing amounts of PVP will lead to loss of acceptable permeation rates for delivery of a therapeutic level of drug and desired adhesive properties.

The invention evolved from the discovery that the transdermal permeation rate of a drug from the pressure-sensitive adhesive system can be selectively modulated by adjusting the solubility of the drug in the system. As used herein, the term "transdermal permeation rate" means the rate of passage of the drug through the skin; which, as known in the art, may or may not be affected by the rate of release of the drug from the carrier.

Forming a blend of multiple polymers results in an adhesive system having a characteristic "net solubility parameter," the selection of which advantageously permits a selectable modulation of the delivery rate of the drug by adjusting the solubility of the drug in the multiple polymer adhesive system.

Solubility parameter, also referred to herein as "SP," has been defined as the sum of all the intermolecular attractive forces, which are empirically related to the extent of mutual solubility of many chemical species. A general discussion of solubility parameters is found in an article by Vaughan, "Using Solubility Parameters in Cosmetics Formulation," J. Soc. Cosmet. Chem., Vol.36, pages 319-333 (1985).

The multiple polymer adhesive system is preferably formulated so that it is a pressure-sensitive adhesive at room temperature and has other desirable characteristics for adhesives used in the transdermal drug delivery art. Such characteristics include good adherence to skin, ability to be peeled or otherwise removed without substantial trauma to the skin, retention of tack with aging, etc. In general, the multiple polymer adhesive system should have a glass transition temperature (T_{g}), measured using a differential scanning calorimeter, of between about -70°C and 0°C.

The term "acrylic polymer" is used herein as in the art interchangeably with polyacrylate, polyacrylic and acrylic adhesive. The acrylic-based polymer and silicone-based polymer are preferably in a ratio by weight, respectively, from about 2:98 to about 96:4, more preferably from about 2:98 to about 90:10, and even more preferably about 2:98 to about 86:14. The amount of acrylic-based (hereinafter referred to broadly as a polyacrylate) polymer and silicone-based polymer (hereinafter referred to broadly as a polysiloxane) is selected to modify the saturation concentration of the drug in the ternary multiple polymer adhesive system in order to affect the rate of delivery of the drug from the system and through the skin.

In particularly improved embodiments of the invention, the polyacrylate is present in an amount ranging from about 5 - 85% by weight of the pressure-sensitive adhesive composition and polyisobutylene is present in an amount ranging from about 14 - 94% by weight of the total composition. In yet another preferred embodiment, a polyisobutylene is present in an amount ranging from about 10 - 90% by weight of the total composition and the polyacrylate is present in an amount ranging from about 5 - 85% by weight of the total composition.

The concentration by weight of the drug in the pressure-sensitive adhesive composition is preferably about 0.1% to about 50%, more preferably about 0.1% to about 40%, and optimally about 0.3% to about 30%, said percentages being based on the total weight of the pressure-sensitive adhesive composition. The invention is especially useful for drugs to be used in low concentrations, *e.g.* 10%, 5% or even 3% of the composition. Irrespective of whether there is high-loading or low-loading of the drug into the transdermal drug delivery system, the pressure-sensitive adhesive composition of the present invention can be formulated to maintain acceptable shear, tack, and peel adhesive properties.

In the practice of preferred embodiments of the invention, the polyacrylate can be any of the homopolymers, copolymers, terpolymers, and the like of various acrylic acids. In such preferred embodiments, the polyacrylate constitutes preferably up to about 85% of the total weight of the pressure-sensitive adhesive composition, more preferably about 3% to about 85%, and optimally about 5% to about 85%, the amount of polyacrylate being dependent on the amount and type of drug used.

The polyacrylates useful in practicing the invention are polymers of one or more monomers of acrylic acids and other copolymerizable monomers. The polyacrylates also include copolymers of alkyl acrylates and/or methacrylates and/or copolymerizable secondary monomers or monomers with functional groups. By varying the amount of each type of monomer added, the cohesive properties of the resulting polyacrylate can be changed as is known in the art. In general, the polyacrylate is composed of at least 50% by weight of an acrylate or alkyl acrylate monomer, from 0 to 20% of a functional monomer copolymerizable with the acrylate, and from 0 to 40% of other monomers.

Further details and examples of acrylic adhesives which are suitable in the practice of the invention are described in Satas, "Acrylic Adhesives," Handbook of Pressure-sensitive Adhesive Technology, 2nd ed., pp. 396-456 (D. Satas, ed.), Van Nostrand Reinhold, New York (1989).

Suitable acrylic adhesives are commercially available and include the polyacrylate adhesives sold under the trademarks Duro-Tak 80-1194, 80-1196, 80-1197, 2287, 2516 and 2852 by National Starch and Chemical Corporation, Bridgewater, New Jersey. Other suitable acrylic adhesives are those sold under the trademarks Gelva-Multipolymer Solution GMS 737, 788, 1151 and 1430 (Monsanto; St. Louis, MO).

The rubber adhesives useful in practicing the invention include hydrocarbon polymers such as natural and synthetic polyisoprene, polybutylene and polyisobutylene, styrene/butadiene polymers, styrene-isoprene-styrene block copolymers, hydrocarbon polymers such as butyl rubber, halogen-containing polymers such as polyacrylo-nitrile, polytetrafluoroethylene, polyvinylchloride, polyvinylidene chloride, and polychloropiene, and polysiloxanes and other copolymers thereof.

Suitable polysiloxanes include silicone pressure-sensitive adhesives which are based on two major components: a polymer, or elastomer, and a tackifying resin. The polysiloxane adhesive is usually prepared by cross-linking the elastomer, typically a high molecular weight polydiorganosiloxane, with the resin, to produce a three-dimensional siloxane structure, via a condensation reaction in an appropriate organic solvent. The ratio of resin to elastomer is the most important factor which can be adjusted in order to modify the physical properties of polysiloxane adhesives. Sobieski, *et al.*, "Silicone Pressure Sensitive Adhesives," Handbook of Pressure-Sensitive Adhesive Technology, 2nd ed., pp. 508-517 (D. Satas, ed.), Van Nostrand Reinhold, New York (1989).

Further details and examples of silicone pressure-sensitive adhesives which are useful in the practice of this invention are described in the following U.S. Patents: 4,591,622; 4,584,355; 4,585,836; and 4,655,767.

Suitable silicone pressure-sensitive adhesives are commercially available and include the silicone adhesives sold under the trademarks BIO-PSA X7-3027, X7-4203, Q7-4503, X7-4603, X7-4301, X7-4303, X7-4919, X7-2685, and X7-3122 by Dow Corning Corporation, Medical Products, Midland, Michigan. BIO-PSA X7-4203, X7-4301 and X7-4303 are particularly suitable for use in formulations containing amine-functional drugs, such as albuterol.

In the practice of preferred embodiments of the invention, the polysiloxane constitutes preferably from about 9% to about 97% of the total weight of the pressure-sensitive adhesive composition, more preferably about 12% to about 97%, and optimally about 14% to about 94%.

Drugs in general can be used in this invention. These drugs include those categories and species of drugs set forth on page ther-5 to ther-29 of the Merck Index, 11th Edition Merck & Co. Rahway, N.J. (1989). Exemplary of drugs that can be administered by the novel transdermal drug delivery system of this invention include, but are not limited to:
1. β-Adrenergic agonists such as Albuterol, Bambuterol, Bitolterol, Carbuterol, Clenbuterol, Clorprenaline, Denopamine, Dioxethedrine, Dopexamine, Ephedrine, Epinephrine, Etafedrine, Ethylnorepinephrine, Fenoterol, Formoterol, Hexoprenaline, Ibopamine, Isoetharine, Isoproterenal, Mabuterol, Metaproterenol, Methoxyphenamine, Oxyfedrine, Pirbuterol, Prenalterol, Procaterol, Protokylol, Reproterol, Rimiterol, Ritodrine, Soterenol, Terbutaline, Terbuterol and Xamoterol.
2. β-Adrenergic blockers such as Acebutolol, Alprenolol, Amosulalol, Arotinolol, Atenolol, Befunolol, Betaxolol, Bevantolol, Bisoprolol, Bopindolol, Bucumolol, Befetolol, Bufuralol, Bunitrolol, Bupranolol, Butidrine Hydrochloride, Butofilolol, Carazolol, Cartezolol, Carvedilol, Celiprolol, Cetamolol, Cloranolol, Dilevalol, Epanolol, Esmolol, Indenolol, Labetalol, Levobunolol, Mepindolol, Metipranalol, Metoprolol, Moprolol, Nadoxolol, Nifenalol, Nipradilol, Oxprenolol, Penbutolol, Pindolol, Practolol, Pronethalol, Propranolol, Sotalol, Sulfinalol, Talinolol, Tertatolol, Timolol, Toliprolol and Xibenolol.
3. Analgesics such as Chlorobutanol; Narcotics such as Alfentanil, Allylprodine, Alphaprodine, Anileridine, Benzylmorphine, Bezitramide, Buprenorphine, Butorphanol, Clonitazene, Codeine, Codeine Methyl Bromide, Codeine Phosphate, Codeine Sulfate, Desomorphine, Dextromoramide, Dezocine, Diampromide, Dihydrocodeine, Dihydrocodeinone Enol Acetate, Dihydromorphine, Dimenoxadol, Dimepheptanol, Dimethylthiambutene, Dioxaphetyl Butyrate, Dipipanone, Eptazocine, Ethoheptazine, Ethylmethlythiambutene, Ethylmorphine, Etonitazene, Fentanyl, Hydrocodone, Hydromorphone, Hydroxypethidine, Isomethadone, Ketobemidone, Levorphanol, Lofentanil, Meperidine, Meptazinol, Metazocine, Methadone Hydrochloride, Metopon, Morphine, Morphine Derivatives, Myrophine, Nalbuphine, Narceine, Nicomorphine, Norlevorphanol, Normethadone, Normorphine, Norpipanone, Opium, Oxycodone, Oxymorphone, Papaveretum, Pentazocine, Phenadoxone, Phenazocine, Pheoperidine, Piminodine, Piritramide, Proheptazine, Promedol, Properidine, Propiram, Propoxyphene, Sufentanil and Tilidine and non-Narcotics such as Acetaminophen, Acetylsalicylsalicylic Acid and Alclofenac.
4. Anesthetics such as Lidocaine, Tetracaine, Dyclonine, Dibucaine, Procaine, Mepivacaine, Bupivacaine, Etidocaine, Prilocaine and Benzocaine.
5. Antianginals such as Acebutolol, Alprenolol, Amiodarone, Amlodipine, Arotinolol, Atenolol, Bepridil, Bevantolol, Bucumolol, Bufetolol, Bufuralol, Bunitrolol, Bupranolol, Carozolol, Carteolol, Carvedilol, Celiprolol, Cinepazet Maleate, Diltiazem, Epanolol, Felodipine, Gallopamil, Imolamine, Indenolol, Isosorbide Dinitrate, Isosorbide Mononitrate, Isradipine, Limaprost, Mepindolol, Metoprolol, Molsidomine, Nadolol, Nicardipine, Nifedipine, Nifenalol, Nilvadipine, Nipradilol, Nisoldipine, Nitroglycerin, Oxprenolol, Oxyfedrine, Ozagrel, Penbutolol, Pentaerythritol Tetranitrate, Pindolol, Pronethalol, Propranolol, Sotalol, Terodiline, Timolol, Toliprolol and Verapamil.
6. Antiarrhythmics such as Acebutol, Acecaine, Adenosine, Ajmaline, Alprenolol, Amiodarone, Amoproxan, Aprindine, Arotinolol, Atenolol, Bevantolol, Bretylium Tosylate, Bubumolol, Bufetolol, Bunaftine, Bunitrolol, Bupranolol, Butidrine Hydrochloride, Butobendine, Capobenic Acid, Carazolol, Carteolol, Cifenline, Cloranolol, Disopyramide, Encainide, Esmolol, Flecainide, Gallopamil, Hydroquinidine, Indecainide, Indenolol, Ipratropium Bromide, Lorajmine, Lorcainide, Meobentine, Metipranolol, Mexiletine, Moricizine, Nadoxolol, Nifenalol, Oxprenolol, Penbutolol, Pindolol, Pirmenol, Practolol, Prajmaline, Procainamide Hydrochloride, Pronethalol, Propafenone, Propranolol, Pyrinoline, Quinidine Sulfate, Quinidine, Sotalol, Talinolol, Timolol, Tocainide, Verapamil, Viquidil and Xibenolol.
7. Antidepressants, including:
   Bicyclics such as Binedaline, Caroxazone, Citalopram, Dimethazan, Indalpine, Fencamine, Indeloxazine Hydrochcloride, Nefopam, Nomifensine, Oxitriptan, Oxypertine, Paroxetine, Sertraline, Thiazesim, Trazodone and Zometapine;
   Hydrazides/Hydrazines such as Benmoxine, Iproclozide, Iproniazid, Isocarboxazid, Nialamide, Octamoxin and Phenelzine;
   Pyrrolidones such as Cotinine, Rolicyprine and Rolipram;
   Tetracyclics such as Maprotiline, Metralindole, Mianserin and Oxaprotiline.
   Tricyclics such as Adinazolam, Amitriptyline, Amitriptylinoxide, Amoxapine, Butriptyline, Clomipramine, Demexiptiline, Desipramine, Dibenzepin, Dimetracrine, Dothiepin, Doxepin, Fluacizine, Imipramine, Imipramine *N*-Oxide, Iprindole, Lofepramine, Melitracen, Metapramine, Nortriptyline, Noxiptilin, Opipramol, Pizotyline, Propizepine, Protriptyline, Quinupramine, Tianeptine and Trimipramine; and
   Others such as Adrafinil, Benactyzine, Bupropion, Butacetin, Deanol, Deanol Aceglumate, Deanol Acetamidobenzoate, Dioxadrol, Etoperidone, Febarbamate, Femoxetine, Fenpentadiol, Fluoxetine, Fluvoxamine, Hematoporphyrin, Hypercinin, Levophacetoperane, Medifoxamine, Minaprine, Moclobemide, Oxaflozane, Piberaline, Prolintane, Pyrisuccideanol, Rubidium Chloride, Sulpiride, Sultopride, Teniloxazine, Thozalinone, Tofenacin, Toloxatone, Tranylcypromine, L-Tryptophan, Viloxazine and Zimeldine.
8. Antiestrogens such as Delmadinone Acetate, Ethamoxytriphetol, Tamoxifen and Toremifene.
9. Antigonadotropins such as Danazol, Gestrinone and Paroxypropione.
10. Antihypertensive drugs, including:
   Benzothiadiazine derivatives such as Althiazide, Bendroflumethiazide, Benzthiazide, Benzylhydrochlorothiazide, Buthiazide, Chlorothiazide, Chlorthalidone, Cyclopenthiazide, Cyclothiazide, Diazoxide, Epithiazide, Ethiazide, Fenquizone, Hydrochlorothiazide, Hydroflumethiazide, Methyclothiazide, Meticrane, Metolazone, Paraflutizide, Polythiazide, Tetrachlormethiazide and Trichlormethiazide;
   N-Carboxyalkyl (peptide/lactam) derivatives such as Alacepril, Captopril, Cilazapril, Delapril, Enalapril, Enalaprilat, Fosinopril, Lisinopril, Moveltipril, Perindopril, Quinapril and Ramipril;
   Dihydropyridine derivatives such as Amlodipine, Felodipine, Isradipine, Nicardipine, Nifedipine, Nilvadipine, Nisoldipine and Nitrendipine;
   Guanidine derivatives such as Bethanidine, Debrisoquin, Guanabenz, Guanacline, Guanadrel, Guanazodine, Guanethidine, Guanfacine, Guanochlor, Guanoxabenz and Guanoxan;
   Hydrazines and phthalazines such as Budralazine, Cadralazine, Dihydralazine, Endralazine, Hydracarbazine, Hydralazine, Pheniprazine, Pildralazine and Todralazine;
   Imidazole derivatives such as Clonidine, Lofexidine, Phentolamine, Tiamenidine and Tolonidine;
   Quaternary ammonium compounds Azamethonium Bromide, Chlorisondamine Chloride, Hexamethonium, Pentacynium Bis(methyl sulfate), Pentamethonium Bromide, Pentolinium Tartate, Phenactopinium Chloride and Trimethidiunum Methosulfate;
   Quinazoline derivatives such as Alfuzosin, Bunazosin, Doxazosin, Prasosin, Terazosin and Trimazosin;
   Reserpine derivatives such as Bietaserpine, Deserpidine, Rescinnamine, Reserpine and Syrosingopine;
   Sulfonamide derivatives such as Ambuside, Clopamide, Furosemide, Indapamide, Quinethazone, Tripamide and Xipamide; and
   Others such as Ajmaline, γ-Aminobutyric Acid, Bufeniode, Chlorthalidone, Cicletaine, Ciclosidomine, Cryptenamine Tannates, Fenoldopam, Flosequinan, Indoramin, Ketanserin, Metbutamate, Mecamylamine, Methyldopa, Methyl 4-Pyridyl Ketone Thiosemicarbarzone, Metolazone, Minoxidil, Muzolimine, Pargyline, Pempidine, Pinacidil, Piperoxan, Primaperone, Protoveratrines, Raubasine, Rescimetol, Rilmenidene, Saralasin, Sodium Nitroprusside, Ticrynafen, Trimethaphan Camsylate, Tyrosinase and Urapidil.
11. Anti-Inflammatory (non-steroidal) drugs, including:
   Aminoarylcarboxylic acid derivatives such as Enfenamic Acid, Etofenamate, Flufenamic Acid, Isonixin, Meclofenamic Acid, Mefanamic Acid, Niflumic Acid, Talniflumate, Terofenamate and Tolfenamic Acid;
   Arylacetic acid derivatives such as Acemetacin, Alclofenac, Amfenac, Bufexamac, Cinmetacin, Clopirac, Diclofenac Sodium, Etodolac, Felbinac, Fenclofenac, Fenclorac, Fenclozic Acid, Fentiazac, Glucametacin, Ibufenac, Indomethacin, Isofezolac, Isoxepac, Lonazolac, Metiazinic Acid, Oxametacine, Proglumetacin, Sulindac, Tiaramide, Tolmetin and Zomepirac;
   Arylbutyric acid derivatives such as Bumadizon, Butibufen, Fenbufen and Xenbucin;
   Arylcarboxylic acids such as Clidanac, Ketorolac and Tinoridine;
   Arylpropionic acid derivatives such as Alminoprofen, Benoxaprofen, Bucloxic Acid, Carprofen, Fenoprofen, Flunoxaprofen, Flurbiprofen, Ibuprofen, Ibuproxam, Indoprofen, Ketoprofen, Loxoprofen, Miroprofen, Naproxen, Oxaprozin, Piketoprofen, Pirprofen, Pranoprofen, Protizinic Acid, Suprofen and Tiaprofenic Acid;
   Pyrazoles such as Difenamizole and Epirizole;
   Pyrazolones such as Apazone, Benzpiperylon, Feprazone, Mofebutazone, Morazone, Oxyphenbutazone, Phenybutazone, Pipebuzone, Propyphenazone, Ramifenazone, Suxibuzone and Thiazolinobutazone;
   Salicylic acid derivatives such as Acetaminosalol, Aspirin, Benorylate, Bromosaligenin, Calcium Acetylsalicylate, Diflunisal, Etersalate, Fendosal, Gentisic Acid, Glycol Salicylate, Imidazole Salicylate, Lysine Acetylsalicylate, Mesalamine, Morpholine Salicylate, 1-Naphthyl Salicylate, Olsalazine, Parsalmide, Phenyl Acetylsalicylate, Phenyl Salicylate, Salacetamide, Salicylamine *O*-Acetic Acid, Salicylsulfuric Acid, Salsalate and Sulfasalazine;
   Thiazinecarboxamides such as Droxicam, Isoxicam, Piroxicam and Tenoxicam; and
   Others such as ε-Acetamidocaproic Acid, *S*-Adenosylmethionine, 3-Amino-4-hydroxybutyric Acid, Amixetrine, Bendazac, Benzydamine, Bucolome, Difenpiramide, Ditazol, Emorfazone, Guaiazulene, Nabumetone, Nimesulide, Orgotein, Oxaceprol, Paranyline, Perisoxal, Pifoxime, Proquazone, Proxazole and Tenidap.
12. Antineoplastic drugs, including:
   Alkylating agents, including:
      Alkyl sulfonates such as Busulfan, Improsulfan and Piposulfan;
      Aziridines such as Benzodepa, Carboquone, Meturedepa and Uredepa;
      Ethylenimines and methylmelamines such as Altretamine, Triethylenemelamine, Triethylenephosphoramide, Triethylenethiophosphoramide and Trimethylolomelamine;
      Nitrogen mustards such as Chlorambucil, Chlornaphazine, Chclophosphamide, Estramustine, Ifosfamide, Mechlorethamine, Mechlorethamine Oxide Hydrochloride, Melphalan, Novembichin, Phenesterine, Prednimustine, Trofosfamide and Uracil Mustard;
      Nitrosoureas such as Carmustine, Chlorozotocin, Fotemustine, Lomustine, Nimustine and Ranimustine; and
      Others such as Dacarbazine, Mannomustine, Mitobronitol, Mitolactol and Pipobroman;
      Antibiotics such as Aclacinomycins, Actinomycin F₁, Anthramycin, Azaserine, Bleomycins, Cactinomycin, Carubicin, Carzinophilin, Chromomycins, Dactinomycin, Daunorubicin, 6-Diazo-5-oxo-L-norleucine, Doxorubicin, Epirubicin, Mitomycins, Mycophenolic Acid, Nogalamycin, Olivomycins, Peplomycin, Plicamycin, Porfiromycin, Puromycin, Streptonigrin, Streptozocin, Tubercidin, Ubenimex, Zinostatin and Zorubicin;
   Antimetabolites, including:
      Folic acid analogs such as Denopterin, Methotrexate, Pteropterin and Trimetrexate;
      Purine analogs such as Fludarabine, 6-Mercaptopurine, Thiamiprine and Thioguanaine; and
      Pyrimidine analogs such as Ancitabine, Azacitidine, 6-Azauridine, Carmofur, Cytarabine,
      Doxifluridine, Enocitabine, Floxuridine, Fluroouracil and Tegafur;
      Enzymes such as L-Asparaginase; and
      Others such as δ-Amino-levulinic acid Aceglatone, Amsacrine, Bestrabucil, Bisantrene, Carboplatin, Cisplatin, Defofamide, Demecolcine, Diaziquone, Elfornithine, Elliptinium Acetate, Etoglucid, Etoposide, Gallium Nitrate, Hydroxyurea, Interferon-α, Interferon-β, Interferon-γ, Interleukine-2, Lentinan, Lonidamine, Mitoguazone, Mitoxantrone, Mopidamol, Nitracrine, Pentostatin, Phenamet, Pirarubicin, Podophyllinicc Acid, 2-Ethythydrazide, Procarbazine, PSK®, Razoxane, Sizofiran, Spirogermanium, Taxol, Teniposide, Tenuazonic Acid, Triaziquone, 2.2'.2"-Trichlorotriethylamine, Urethan, Vinblastine, Vincristine and Vindesine.
13. Antineoplastic (hormonal) drugs, including:
   Androgens such as Calusterone, Dromostanolone Propionate, Epitiostanol, Mepitiostane and Testolactone;
   Antiadrenals such as Aminoglutethimide, Mitotane and Trilostane;
   Antiandrogens such as Flutamide and Nilutamide; and
   Antiestrogens such as Tamoxifen and Toremifene.
14. Antiparkinsonian drugs such as Amantadine, Benserazide, Bietanautine, Biperiden, Bromocriptine, Budipine, Carbidopa, Deprenyl, Dexetimide, Diethazine, Droxidopa, Ethopropazine, Ethylbenzhydramine, Levodopa, Naxagolide, Pergolide, Piroheptine, Pridinol, Prodipine, Selegiline, Terguride, Tigloidine and Trihexyphenidyl Hydrochloride.
15. Antiprostatic hypertrophy drugs such as Gestonorone Caproate, Mepartricin, Oxendolone and Proscar®.
16. Antipsychotic drugs, including:
   Butyrophenones such as Benperidol, Bromperidol, Droperidol, Fluanisone, Haloperidol, Melperone, Moperone, Pipamperone, Sniperone, Timiperone and Trifluperidol;
   Phenothiazines such as Acetophenazine, Butaperazine, Carphenazine, Chlorproethazine, Chlorpromazine, Clospirazine, Cyamemazine, Dixyrazine, Fluphenazine, Imiclopazine, Mepazine, Mesoridazine, Methoxypromazine, Metofenazate, Oxaflumazine, Perazine, Pericyazine, Perimethazine, Perphenazine, Piperacetazine, Pipotiazine, Prochlorperazine, Promazine, Sulforidazine, Thiopropazate, Thioridazine, Trifluoperazine and Triflupromazine;
   Thioxanthenes such as Chlorprothixene, Clopenthixol, Flupentixol and Thiothixene;
   Tricyclics such as Benzquinamide, Carpipramine, Clocapramine, Clomacran, Clothiapine, Clozapine, Opipramol, Prothipendyl, Tetrabenazine, and Zotepine; and
   Others such as Alizapride, Amisulpride, Buramate, Fluspirilene, Molindone, Penfluridol, Pimozide, Spirilene and Sulpiride.
17. Antispasmodic drugs such as Alibendol, Ambucetamide, Aminopromazine, Apoatropine, Bevonium Methyl Sulfate, Bietamiverine, Butaverine, Butropium Bromide, *N*-Butylscopolammonium Bromide, Caroverine, Cimetropium Bromide, Cinnamedrine, Clebopride, Coniine Hydrobromide, Coniine Hydrochloride, Cyclonium Iodide, Difemerine, Diisopromine, Dioxaphetyl Butyrate, Diponium Bromide, Drofenine, Emepronium Bromide, Ethaverine, Feclemine, Fenalamide, Fenoverine, Fenpiprane, Fenpiverinium Bromide, Fentonium Bromide, Flavoxate, Flopropione, Gluconic Acid, Guaiactamine, Hydramitrazine, Hymecromone, Leiopyrrole, Mebeverine, Moxaverine, Nafiverine, Octamylamine, Octaverine, Pentapiperide, Phenamacide Hydrochloride, Phloroglucinol, Pinaverium Bromide, Piperilate, Pipoxolan Hydrochloride, Pramiverin, Prifinium Bromide, Properidine, Propivane, Propyromazine, Prozapine, Racefemine, Rociverine, Spasmolytol, Stilonium Iodide, Sultroponium, Tiemonium Iodide, Tiquizium Bromide, Tiropramide, Trepibutone, Tricromyl, Trifolium, Trimebutine, *N*,*N*-1-Trimethyl-3,3-diphenylpropylamine, Tropenzile, Trospium Chloride and Xenytropium Bromide.
18. Anxiolytic drugs, including:
   Arylpiperazines such as Buspirone, Gepirone and Ipsapirone;
   Benzodiazepine derivatives such as Alprazolam, Bromazepam, Camazepam, Chlordiazepoxide, Clobazam, Clorazepate, Chotiazepam, Cloxazolam, Diazepam, Ethyl Loflazepate, Etizolam, Fluidazepam, Flutazolam, Flutoprazepam, Halazepam, Ketazolam, Lorazepam, Loxapine, Medazepam, Metaclazepam, Mexazolam, Nordazepam, Oxazepam, Oxazolam, Pinazepam, Prazepam, Tofisopam and Triazolam;
   Carbamates such as Cyclarbamate, Emylcamate, Hydroxyphenamate, Meprobamate, Phenprobamate and Tybamate; and
   Others such as Alpidem, Benzoctamine, Captodiamine, Chlormezanone, Clonazepam, Etifoxine, Flurazepam, Fluoresone, Glutamic Acid, Hydroxyzine, Mecloralurea, Mephenoxalone, Oxanamide, Phenaglycodol, Suriclone.
19. Benzodiazepine antagonists such as Flumazenil.
20. Bronchodilators, including:
   Ephedrine derivatives such as Albuterol, Bambuterol, Bitolterol, Carbuterol, Clenbuterol, Clorprenaline, Dioxethedrine, Ephedrine, Epiniphrine, Eprozinol, Etafedrine, Ethylnorepineghrine, Fenoterol, Hexoprenaline, Isoetharine, Isoproterenol, Mabuterol, Metaproterenol, *N*-Methylephedrine, Pirbuterol, Procaterol, Protokylol, Reproterol, Rimiterol, Soterenol, Terbutaline and Tulobuterol;
   Quaternary ammonium compounds such as Bevonium Methyl Sulfate, Clutropium Bromide, Ipratropium Bromide and Oxitropium Bromide;
   Xanthine derivatives such as Acefylline, Acefylline Piperazine, Ambuphylline, Aminophylline, Bamifylline, choline Theophyllinate, Doxofylline, Dyphylline, Enprofylline, Etamiphyllin, Etofylline, Guaithylline, Proxyphylline, Theobromine, 1-Theobromineacetic Acid and Theophylline; and
   Others such as Fenspiride, Medibazine, Methoxyphenanime and Tretoquinol.
21. Calcium regulators such as Calcifediol, Calcitonin, Calcitriol, Clodronic Acid, Dihydrotachysterol, Elcatonin, Etidronic Acid, Ipriflavone, Pamidronic Acid, Parathyroid Hormone and Teriparatide Acetate.
22. Cardiotonics such as Acefylline, Acetyldigititoxins, 2-Amino-4-picoline, Amrinone, Benfurodil Hemisuccinate, Buclasdesine, Cerberoside, Camphotamide, Convallatoxin, Cymarin, Denopamine, Deslanoside, Ditalin, Digitalis, Digitoxin, Digoxin, Dobutamine, Dopamine, Dopexamine, Enoximone, Erythrophleine, Fenalcomine, Gitalin, Gitoxin, Glycocyamine, Heptaminol, Hydrastinine, Ibopamine, Lanotodises, Metamivam, Milrinone, Neriifolin, Oleandrin, Ouabain, Oxyfedrine, Prazosin, Prenalterol, Proscillaridin, Resibufogenin, Scillaren, Scillarenin, Strophanthin, Sulmazole, Theobromine and Xamoterol.
23. Chelating agents such as Deferozmine, Ditiocarb Sodium, Edetate Calcium Disodium, Edetate Disodium, Edeate Sodium, Edetate Trisodium, Penicillamine; Pentetate Calcium Trisodium, Pentectic Acid, Succimer and Trientine.
24. Cholinergic agonists such as choline, acetylcholine, metacholine, carbachol, bethanechol, pilocarpine, muscarine and arecoline.
25. Dopamine receptor agonists such as Bromocriptine, Dopexamine, Fenoldopam, Ibopamine, Lisuride, Naxagolide and Pergolide.
26. Enzyme inducers (hepatic) such as Flumecinol.
27. Estrogens, including:
   Nonsteroidal estrogens such as Benzestrol, Broparoestrol, Chlorotrianisene, Dienestrol, Diethylstilbestrol, Diethylstilbestrol Diproprionate, Dimestrol, Fosfestrol, Hexestrol, Methallenestril and Methestrol; and
   Steroidal estrogens such as Colpormon, Conjugated Estrogenic Hormones, Equilenin, Equilin, Esterfied Estrogens, Estradiol, Estradiol Benzoate, 17β-Estradiol, Estradiol 17β-Cypionate, Estriol, Estrone, Estropipate, Ethinyl Estradiol, Ethinylestrenol, Mestranol, Moxestrol, Mytatrienediol, Quinestradiol and Quinestrol.
28. Glucocorticoids such as 21-Acetoxyprefnenolone, Aalclometasone, Algestone, Amicinonide, Beclomethasone, Betamethasone, Budesonide, Chloroprednisone, Clobetasol, Blovetasone, Clocortolone, Cloprednol, Corticosterone, Cortisone, Cortivazol, Deflazacort, Desonide, Desoximetasone, Dexamethasone, Diflorasone, Diflucortolone, Difluprednate, Enoxolone, Fluazacort, Flucloronide, Flumehtasone, Flunisolide, Fluocinolone Acetonide, Fluocinonide, Fluocortin Butyl, Fluocortolone, Fluorometholone, Fluperolone Acetate, Fluprednidene Acetate, Fluprednisolone, Flurandrenolide, Formocortal, Halcinonide, Halometasone, Halopredone Acetate, Hydrocortamate, Hydrocortisone, Hydrocortisone Acetate, Hydrocortisone Phosphate Hydrocortisone 21-Sodium Succinate, Hydrocortisone Tebutate, Mazipredone, Medrysone, Meprednisone, Methyolprednisolone, Mometasone Furoate, Paramethasone, Prednicarbate, Prednisolone, Prednisolone 21-Diethylaminoacetate, Prednisone Sodium Phosphate, Prednisolone Sodium Succinate, Prednisolone Sodium 21-*m*-Sulfobenzoate, Prednisolone 21-Stearoylglycolate, Prednisolone Tebutate, Prednisolone 21-Trimethylacetate, Prednisone, Prednival, Prednylidene, Prednylidene 21-Diethylaminoacetate, Tixocortal, Triamcinolone, Triamcinolone Acetonide, Triamcinolone Benetonide and Triamcinolone Hexacetonide.
29. Mineralcorticoids such as Aldosterone, Deoxycorticosterone, Deoxycorticosterone Acetate and Fludrocortisone.
30. Monoamine oxidase inhibitors such as Deprenyl, Iproclozide, Iproniazid, Isocarboxazid, Moclobemide, Octomoxin, Pargyline, Phenelzine, Phenoxypropazine, Pivalylbenzhydrazine, Prodipine, Toloxatone and Tranylcypromine.
31. Muscle relaxants (skeletal) such as Afloqualone, Alcuronium, Atracurium Besylate, Baclofen, Benzoctamine, Benzoquinonium Chloride, *C*-Calebassine, Carisoprodol, Chlormezanone, Chlorphenesin Carbamate, Chlorproethazine, Chlozoxazone, Curare, Cyclarbamate, Cyclobenzaprine, Dantrolene, Decamethonium Bromide, Diazepam, Eperisone, Fazadinium Bromide, Flumetramide, Gallamine Triethiodide, Hexacarbacholine Bromide, Hexafluorenium Bromide, Idrocilamide, Lauexium Methyl Sulfate,. Leptodactyline, Memantine, Mephenesin, Mephenoxalone, Metaxalone, Methocarbamol, Metocurine Iodide, Nimetazepam, Orphenadrine, Pancuronium Bromide, Phenprobamate, Phenyramidol, Pipecurium Bromide, Promoxolane, Quinine Sulfate, Styramate, Succinylcholine Bromide, Succinylcholine Chloride, Succinylcholine Iodine, Suxethonium Bromide, Tetrazepam, Thiocolchicoside, Tizanidine, Tolperisone, Tubocurarine Chloride, Vecuronium Bromide and Zoxolamine.
32. Narcotic antagonists such as Amiphenazole, Cyclazocine, Levallorphan, Nadide, Nalmfene, Nalorphine, Nalorphine Dinicotinate, Naloxone and Naltrexone.
33. Progestogens such as Allylestrenol, Anagestone, Chlormadinone Acetate, Delmadinone Acetate, Demegestone, Desogestrel, Dimethisterone, Dydrogesterone, Ethisterone, Ethynodiol, Ethynodial Diacetate, Flurogestone Acetate, Gestodene, Gestonorone Caproate, Haloprogesterone, 17-Hydroxy-16-methylene--progesterone, 17α-Hydroxyprogesterone, 17α-Hydroxygesterone Caproate, Hydroxyprogesterone Caproate, Lynestrenol, Medrogestone, Medroxyprogesterone, Medroxygesterone Acetate, Megestrol Acetate, Melengestrol, Norethindrone, Norethindrone Acetate, Norethynodrel, Norgesterone, Norgestimate, Norgestrel, Norgestrienone, 19-Norprogesterone, Norvinisterone, Pentagestrone, Progesterone, Promegestone, Quingestrone and Trengestone and esters thereof.
34. Vasodilators (coronary), such as Amotriphene, Bendazol, Benfurodil Hemisuccinate, Benziodarone, Chloacizine, Chromonar, Clobenfurol, Clonitrate, Dilazep, Dipyridamole, Droprenilamine, Efloxate, Erythritol, Erythrityl Tetranitrate, Etafenone, Fendiline, Floredil, Ganglefene, Hexestrol Bis(β-diethylaminoethyl ether), Hexobendine, Itramin Tosylate, Khellin, Lidoflazine, Mannitol Hexanitrate, Medibazine, Nicorandil, Nitroglycerin, Pentaerythritol Tetranitrate, Pentrinitrol, Perhexiline, Pimefylline, Prenylamine, Propatyl Nitrate, Pyridofylline, Trapidil, Tricromyl, Trimetazidine, Trolnitrate Phosphate and Visnadine and Vasodilators peripheral such as Aluminum Nicotinate, Bamethan, Bencyclane, Betahistine, Bradykinin, Brovincamine, Bufoniode, Buflomedil, Butalamine, Cetiedil, Ciclonicate, Cinepazide, Cinnarizine, Cyclandelate, Diisopropylamine Dichloracetate, Eledoisin, Fenoxidil, Flunarisine, Heronicate, Ifenprodil, Inositol Niacinate, Isoxsuprine, Kallidin, Kallikrein, Moxisylyte, Nafronyl, Nicametate, Nicergoline, Nicofuranose, Nicotine, Nicotinyl Alcohol, Nylidrin, Papaverine, Pentifylline, Pentoxifylline, Piribedil, Protaglandin E₁, Suloctidil and Xanthinal Niacinate.

The drugs and mixtures thereof can be present in the composition in different forms, depending on which form yields the optimum delivery characteristics. Thus, in the case of drugs, the drug can be in its free base or acid form, or in the form of salts, esters, or any other pharmacologically acceptable derivatives, or as components of molecular complexes.

The amount of drug to be incorporated in the composition varies depending on the particular drug, the desired therapeutic effect, and the time span for which the device is to provide therapy. For most drugs, the passage of the drugs through the skin will be the rate-limiting step in delivery. Thus, the amount of drug and the rate of release is typically selected so as to provide transdermal delivery characterized by a zero order time dependency for a prolonged period of time. The minimum amount of drug in the system is selected based on the amount of drug which passes through the skin in the time span for which the device is to provide therapy. Normally, the amount of drug in the system can vary from about 0.1% to about 50% by weight, and optimally, for the lower drug doses permitted by this invention, from about 0.3% to about 30%.

Of course, the composition of the transdermal drug delivery system can also contain agents known to accelerate the delivery of the drug through the skin. These agents have been referred to as skin penetration enhancers, accelerants, adjuvants, and sorption promoters, and are collectively referred to herein as "enhancers." This class of agents includes those with diverse mechanisms of action including those which have the function of improving the solubility and diffusibility of the drug within the multiple polymer and those which improve percutaneous absorption, for example, by changing the ability of the stratum corneum to retain moisture, softening the skin, improving the skin's permeability, acting as penetration assistants or hair-follicle openers or changing the state of the skin including the boundary layer. Some of these agents have more than one mechanism of action, but in essence they serve to enhance the delivery of the drug. An enhancer may be included in a drug delivery system up to about 20% by weight. If an enhancer is included, then the enhancer is preferably present in an amount of about 1% to about 10% by weight. Some examples of enhancers are polyhydric alcohols such as dipropylene glycol, propylene glycol, and polyethylene glycol which enhance drug solubility; oils such as olive oil, squalene, and lanolin; polyethylene glycol ethers and fatty ethers such as cetyl ether and oleyl ether; fatty acid esters such as isopropyl myristate which enhance drug diffusibility; fatty acid alcohols such as oleyl alcohol; urea and urea derivatives such as allantoin which affect the ability of keratin to retain moisture; polar solvents such as dimethyldecylphosphoxide, methyloctylsulfoxide, dimethyllaurylamide, dodecylpyrrolidone, isosorbitol, dimethylacetonide, dimethylsulfoxide, decylmethylsulfoxide, and dimethylformamide which affect keratin permeability; salicylic acid which softens the keratin; amino acids which are penetration assistants; benzyl nicotinate which is a hair follicle opener; and higher molecular weight aliphatic surfactants such as lauryl sulfate salts which change the surface state of the skin and drugs administered. Other agents include oleic and linoleic acids, ascorbic acid, panthenol, butylated hydroxytoluene, tocopherol, tocopheryl acetate, tocopheryl linoleate, propyl oleate, isopropyl palmitate, oleamide, polyoxyethylene (4) lauryl ether, polyoxyethylene (2) oleyl ether and polyoxyethylene (10) oleyl ether sold under the trademarks Brij 30, 93 and 97 by ICI Americas, Inc., and polysorbate 20 sold under the trademark Tween 20 by ICI Americas, Inc.

In certain embodiments of the invention a plasticizer or tackifying agent is incorporated into the formulation to improve the adhesive characteristics of the pressure-sensitive adhesive composition. A tackifying agent is particularly useful in those embodiments in which the drug does not plasticize the polymer. Suitable tackifying agents are those known in the art including: (1) aliphatic hydrocarbons; (2) mixed aliphatic and aromatic hydrocarbons; (3) aromatic hydrocarbons; (4) substituted aromatic hydrocarbons; (5) hydrogenated esters; (6) polyterpenes; and (7) hydrogenated wood resins or rosins. The tackifying agent employed is preferably compatible with the blend of polymers. In preferred embodiments, the tackifying agent is silicone fluid (e.g., 360 Medical Fluid, available from Dow Corning corporation, Midland, MI) or mineral oil. Silicone fluid is useful for blends comprising polysiloxane as a major component. In other embodiments, where a synthetic rubber, for example, is a major component, mineral oil is a preferred tackifying agent. Acrylics can be tackified with oleates, oleic acid, oleyl alcohol and other fatty acid-derived agents.

Some drugs, such as the vasodilator nitroglycerin, function as plasticizers in the composition because they are soluble to a certain degree in the polymers comprising the system. For drug molecules which are not readily soluble in the polymer system, a co-solvent for the drug and polymer can be added. Co-solvents, such as lecithin, retinol derivatives, tocopherol, dipropylene glycol, triacetin, propylene glycol, saturated and unsaturated fatty acids, mineral oil, silicone fluid, alcohols, butyl benzyl phthalate, and the like are useful in the practice of the instant invention depending on the solubility of the drug in the multiple polymer adhesive system.

To summarize, the preferred and optimum compositions for rubber and polyacrylate embodiments are as follows:

**TABLE I**

| PERCENT BY WEIGHT | | |
|---|---|---|
| Component | Preferred Range | Optimum Range |
| Rubber | 94 - 9 | 94 - 14 |
| Polyacrylate | 2 - 85 | 5 - 85 |
| PVP | 1 - 20 | 5 - 15 |
| Co-solvent(s) | 0 - 30 | 0 - 20 |
| Enhancer(s) | 0 - 20 | 0 - 15 |
| Drug(s) | 0.1 - 50 | 0.3 - 30 |

The compositions of this invention may further be provided with various thickeners, fillers and other additives known for use with transdermal drug delivery systems. Where the composition tends to absorb water, for example, when lecithin is used as a co-solvent, hydrophilic substances are especially useful. One type of hydrophilic substance which has been successfully employed is clay. The addition of clay has been found to improve adhesiveness in transdermal formulations without reducing the rate of drug delivery. Suitable clays include kaolinites such as baolinite, anauxite, dickite and nacrite, montmorillonites such as montmorillonite, bentonite, berdellite and montronite, illites/muscovites such as illite and glauconite, chlorites, polygorshites such as attapulgite, halloysite, metabolloysite, allophane and aluminum silicate clays.

In a device aspect of the invention, the pressure-sensitive adhesive composition can be used as an adhesive portion of any transdermal drug delivery system (e.g., a reservoir device) or it can comprise an adhesive monolithic device. Of course, the principles of the invention would still apply to embodiments where the transdermal drug delivery composition is not a pressure-sensitive adhesive and comprises a drug reservoir.

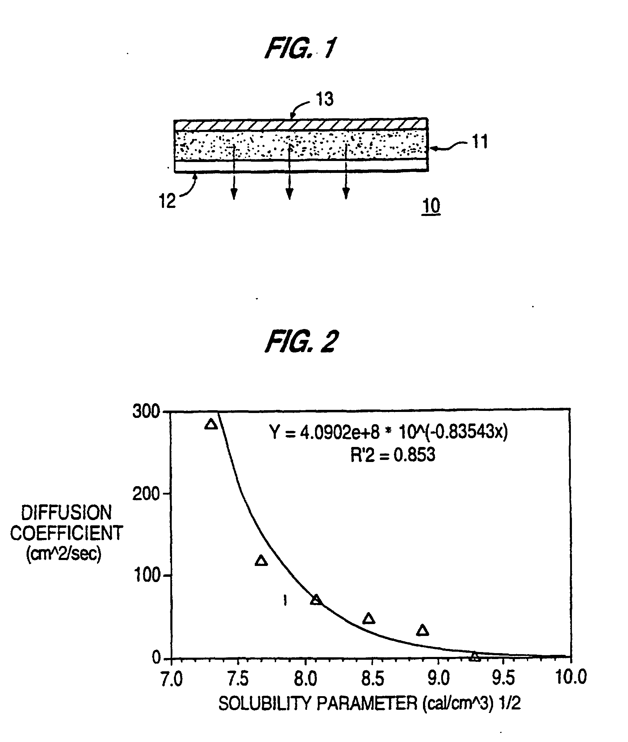

Reference to FIG. 1 shows a schematic illustration of an adhesive monolithic device embodiment of the invention 10. The transdermal drug delivery system comprises a monolithic body 11 of a defined geometric shape with a protective release liner 12 on one side of monolithic body 11 and a backing layer 13 on the other side. Removal of the release liner 12 exposes the pressure-sensitive multiple polymer adhesive composition which functions both as the drug carrier matrix and as the means of applying the system to the patient.

A device, or individual dosage unit, of the present invention can be produced in any manner known to those of skill in the art. After the dermal composition is formed, it may be brought into contact with the backing layer in any manner known to those of skill in the art. Such techniques include calendar coating, hot melt coating, solution coating, *etc.* Of course, backing materials are well known in the art and can comprise plastic films of polyethylene, vinyl acetate resins, polyester, polypropylene, BAREX®, ethylene/vinyl acetate copolymers, polyvinyl chloride, polyurethane, and the like, metal foils, non-woven fabric, cloth, coextrusions or laminations of the above and commercially available laminates. The backing material generally has a thickness in the range of 2 to 1000 micrometers and the dermal composition is generally disposed on backing material in a thickness ranging from about 12 to 250 micrometers thick.

Suitable release liners are also well known in the art and include the commercially available products of Release International designated Bio-Release® liner and Syl-off® 7610 liner. For preferred embodiments in which a polysiloxane is part of the multiple polymer adhesive system, the release liner must be compatible with the silicone adhesive. An example of a suitable commercially available liner is 3M's 1022 ScotchPak.

The configuration of the transdermal delivery system of the present invention can be in any shape or size as is necessary or desirable. Illustratively, a single dosage unit may have a surface area in the range of 1 to 200 cm². Preferred sizes are from 5 to 60 cm².

In a method aspect of the invention, a plurality of polymers having differing solubility parameters are blended (but not chemically reacted or cross-linked) with soluble PVP to result in a pressure-sensitive adhesive composition which controls delivery of an incorporated drug into and through the epidermis. The blending of polymers results in an adjustment of the saturation concentration of the drug in the polymeric system and therefore permits selective modulation of the transdermal drug delivery rate. The term "blending," of course, incorporates choosing the appropriate polymeric components, and the proportions thereof, to achieve the desired effect.

In a preferred embodiment of the invention, a transdermal drug delivery system is prepared by mixing a soluble PVP, polyacrylate, polysiloxane, drug, co-solvent(s), and tackifying agent, if needed, in an appropriate volatile solvent(s), then casting the mixture and removing the solvent(s) by evaporation to form a film.

Suitable volatile solvents include, but are not limited to, alcohols such as isopropanol and ethanol; aromatics such as xylenes and toluene; aliphatics such as hexane, cyclohexane, and heptane; and alkanoic acid esters such as ethyl acetate and butyl acetate.

An exemplary general method of preparation is as follows:
1. Appropriate amounts of soluble PVP, solvent(s), enhancer(s), and organic solvent(s) (for example toluene) are combined and thoroughly mixed together in a vessel.
2. The drug is then added to the mixture and agitation is carried out until the drug is uniformly mixed in.
3. Appropriate amounts of polysiloxane and polyacrylate are then added to the drug mixture, and thoroughly mixed.
4. The formulation is then transferred to a coating operation where it is coated onto a protective release liner at a controlled specified thickness. The coated product is then passed through an oven in order to drive off all volatile processing solvents.
5. The dried product on the release liner is then joined to the backing material and wound into rolls for storage.
6. Appropriate size and shape "systems" are die-cut from the roll material and then pouched.

The order of steps, the amount of the ingredients, and the amount and time of agitation or mixing may be important process variables which will depend on the specific polymers, drug, cosolvents, and enhancers used in the formulation. These factors can be adjusted by those skilled in the art, while keeping in mind the object of providing a uniform product. It is believed that a number of other methods, including changing some of the order of steps, can be carried out and will give desirable results. In addition to having various shapes, the dosage units produces may come in various sizes. A surface area in the range of 1 to 200 square centimeters is contemplated, and the presently preferred sizes are: 5, 10, 15, 20, 30, 30 and 60 square centimeters.

Said PVP preferably has a molecular weight of about 2,000 to 1,100,000, more preferably 2,000 to 1,000,000, even more preferably 5,000 to 100,000, and most preferably 7,000 to 54,000.

Preferred embodiments comprise a soluble PVP with a pressure-sensitive adhesive rubber and a polyacrylate. Particularly preferred blends include blends of a polyacrylate, a polysiloxane and a soluble PVP.

Soluble PVP has been found to be highly effective in solubilization of drugs, in adhesive-type transdermal drug delivery systems according to the invention. In particular, soluble PVP has proved useful in maintaining a norethindrone acetate (NETA) system and an NETA/estradiol system substantially crystal-free. Other specific drugs for which soluble PVP is particularly usefully employed according to the invention include albuterol, estradiol, haloperidol and alprazolam.

The amount and type of soluble PVP required in the foregoing preferred embodiment will depend on the quantity and type of drug present in the adhesive, as well as the type of adhesive. For example, the addition of a rubber adhesive to a mixture of a drug and a polyacrylate adhesive will cause a decrease in drug solubility and subsequent drug crystallization as a result of exceeding saturation. However, the addition of soluble PVP to the mixture can increase the apparent solubility of the drug. In other words, the presence of the rubber adhesive decreases the solubilizable drug load of the mixture, while the presence of soluble PVP compensates for this negative effect.

Accordingly, the optimal concentration of soluble PVP in a transdermal drug delivery system is the amount of soluble PVP sufficient to compensate for the reduced solubility of a drug caused by the rubber adhesive. Optimal concentrations of soluble PVP can be readily determined through routine experimentation. Typically, the PVP is present in an amount from about 1% to about 20% by weight, more preferably from about 3% to about 15% by weight, and optimally from about 5% to about 15% by weight.

For example, when the drug is norethindrone acetate (NETA), an optimum concentration of about 10% by weight soluble PVP has been found to inhibit NETA crystal formation without adversely affecting NETA flux from a multiple polymer adhesive system (polyacrylate/polysiloxane). When the drug is estradiol, the inclusion of 5 - 10% of soluble PVP in the formulation not only increases the estradiol flux, but increases the total amount of estradiol through the skin. When the drug is albuterol, an optimum concentration has been found to be about 5% by weight.

Large amounts of soluble PVP can cause a decrease in the flux of drug. For example, when the PVP is present in amounts exceeding about 20% by weight, NETA flux begins to decrease.

The soluble PVP employed according to the invention is dissolved together with one or more of the additional polymeric materials of the inventive blend.

The type and quantity of soluble PVP also can have significant effects on the adhesive properties of the finished product. In adhesives with higher shear properties, it is advantageous to include a lower molecular weight soluble PVP, whereas in low shear adhesives, the higher molecular weight soluble PVP's are preferred.

The following specific examples are included as illustrative of pressure-sensitive adhesive compositions and transdermal drug delivery systems, and methods of making same, within the contemplation of the invention. These examples are in no way intended to be limiting of the scope of the invention.

The following commercially available adhesives were used in the blends comprising the multiple polymer adhesive system of the examples:
"Duro-Tak 80-1194, 80-1196, 80-1054, 80-1074, 80-1058, 80-2434, 80-1070, 80-6172, 80-1197, 87-2287, 87-2516, and 87-2852" are trademarks of National Starch and Chemical Corporation, Bridgewater, New Jersey for acrylic adhesives (polyacrylates) in organic solutions.
"BIO-PSA X7-3027, X7-4919, X7-2685, X7-3122, X7-4603, X7-4301, X7-4303, Q7-4503, Q7-4501 and Q7-4502" are trademarks of Dow Corning Corporation, Medical Products, Midland, Michigan for silicone adhesives (polysiloxanes) in organic solutions. BIO-PSA X7-4303 is particularly suitable for use in formulations containing amine-functional drugs, such as albuterol and pilocarpine, in the following examples.
"Gelva-Multipolymer Solution (GMS) 737, 788, 1151, 1753, 1430 and 2480" are trademarks of Monsanto Company, St. Louis, Missouri, for an acrylic adhesive in organic solution.
"Vistanex LM-LS-LC" is a trademark of Exxon Chemical Company, Houston, Texas, for a polyisobutylene polymer with a Flory molecular weight of 42,600 to 46,100.

The aforementioned polymeric adhesives are supplied, or prepared, as solutions wherein the percent solids by weight are as follows:

| Ingredient | Percent Solids |
|---|---|
| BIO-PSA X7-2685 | 50 |
| BIO-PSA X7-3027 | 50 |
| BIO-PSA X7-3122 | 65 |
| BIO-PSA X7 4301 | 60 |
| BIO-PSA X7-4303 | 60 |
| BIO-PSA Q7-4501 | 60 |
| BIO-PSA Q7-4502 | 60 |
| BIO-PSA Q7-4503 | 60 |
| BIO-PSA X7-4603 | 60 |
| BIO-PSA X7-4919 | 50 |
| Duro-Tak 80-1194 | 45 |
| Duro-Tak 80-1196 | 45 |
| Duro-Tak 80-1197 | 45 |
| Duro-Tak 87-2852 | 34 |
| Elvax 40-W | 100 |
| GMS 737 | 32 |
| GMS 788 | 41 |
| GMS 1151 | 40 |
| GMS 1430 | 41 |
| GMS 1753 | 40 |
| Kraton D 1101 | 100 |
| Kraton D 1107 | 100 |
| Kraton G 1657 | 100 |
| Vistanex LM-LS-LC | 100 |

"360 Medical Fluid" is a trademark of Dow Corning Corporation for a polydimethylsiloxane fluid. In certain embodiments of the invention, 360 Medical Fluid is added as a tackifier to improve the adhesive characteristics of the end product.

### EXAMPLE 1

An estradiol-polymer mixture was prepared by combining 1.0 part of estradiol, 6.0 parts of dipropylene glycol, 8.0 parts of oleic acid, 35.0 parts of toluene, 5.0 parts of polyvinylpyrrolidone (Kollidon 30), and 129.03 parts of polyacrylate adhesive (GMS 737) in an appropriate container, and mixing well until the mixture was completely homogeneous. Then 66.67 parts of polysiloxane adhesive (BIO-PSA Q7-4503) were added, and the blend was thoroughly mixed. The resulting composition has the ingredient concentrations on a "dry" basis, that is, after removal of volatile process solvents, given below.

| COMPONENT | PERCENT BY WEIGHT |
|---|---|
| Polysiloxane Adhesive (BIO-PSA Q7-4503) | 40.0 |
| Polyacrylate Adhesive (GMS 737) | 40.0 |
| Oleic Acid | 8.0 |
| Dipropylene Glycol | 6.0 |
| Polyvinylpyrrolidone (Kollidon 30) | 5.0 |
| Estradiol | 1.0 |
| | $\overline{\text{100.0}}$ |

In the following examples, the method of Example 1 was used with the appropriate amounts of starting materials to yield compositions having the following ingredient concentrations.

| | **EXAMPLE 2** | **EXAMPLE 3** |
|---|---|---|
| COMPONENT | PERCENT BY WEIGHT | |
| Polysiloxane Adhesive (BIO-PSA Q7-4503) | 39.0 | 48.0 |
| Polyacrylate Adhesive (GMS 737) | 40.0 | 30.0 |
| Oleic Acid | 8.0 | 6.0 |
| Dipropylene Glycol | 6.0 | 4.0 |
| Polyvinylpyrrolidone (Koilidon 30) | 5.0 | 10.0 |
| Estradiol | 2.0 | 2.0 |
| | 100.0 | 100.0 |

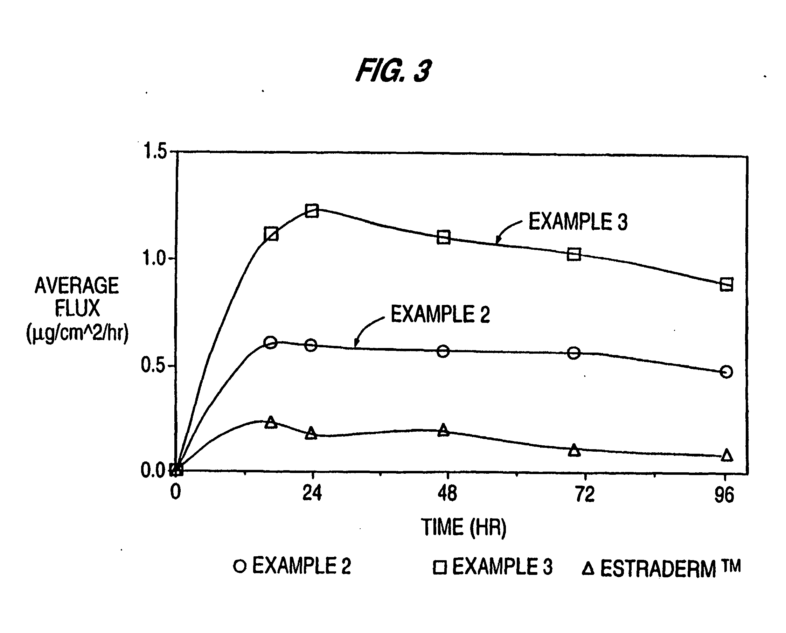

Estradiol permeation through human epidermis in vitro from systems of Examples 2 and 3 are shown in Figure 3. This graph illustrates how the formulas of this invention delivered significantly greater estradiol than Estraderm®, the commercially available estradiol product.

### EXAMPLE 4

An estradiol/norethindrone acetate-polymer mixture was prepared by combining 0.05 parts of estradiol, 3.0 parts of norethindrone acetate, 4.0 parts of dipropylene glycol, 6.0 parts of oleic acid, 10.0 parts of toluene, 10.0 parts of polyvinylpyrrolidone (Kollidon 30), 1.0 parts of butylated hydroxyanisole, and 62.0 parts of polyacrylate adhesive (GMS 737) in an appropriate container, and mixing well until the mixture was completely homogeneous. Then 93.0 parts of polysiloxane adhesive (BIO-PSA Q7-4503) were added, and the blend was thoroughly mixed. The resulting composition has the ingredient concentrations on a "dry" basis, that is, after removal of volatile process solvents, given below.

| COMPONENT | PERCENT BY WEIGHT |
|---|---|
| Polysiloxane Adhesive (BIO-PSA Q7-4503) | 55.95 |
| Polyacrylate Adhesive (GMS 737) | 20.00 |
| Oleic Acid | 6.00 |
| Dipropylene Glycol | 4.00 |
| Polyvinylpyrrolidone (Kollidon 30) | 10.00 |
| Butylated Hydroxyanisole | 1.00 |
| Norethindrone Acetate | 3.00 |
| Estradiol | 0.05 |
| | $\overline{\text{100.00}}$ |

In the following examples, the method of Example 4 was used with the appropriate amounts of starting materials to yield compositions having the following ingredient concentrations.

| **EXAMPLES:** | **5** | **6** | **7** | **8** | **9** | **10** | **11** |
|---|---|---|---|---|---|---|---|
| COMPONENT | PERCENT BY WEIGHT | | | | | | |
| Polysiloxane Adhesive (BIO-PSA Q7-4503) | 55.9 | 55.8 | 55.6 | 55.5 | 55.4 | 55.2 | 55.0 |
| Polyacrylate Adhesive (GMS 737) | 20.0 | 20.0 | 20.0 | 20.0 | 20.0 | 20.0 | 20.0 |
| Oleic Acid | 6.0 | 6.0 | 6.0 | 6.0 | 6.0 | 6.0 | 6.0 |
| Dipropylene Glycol | 4.0 | 4.0 | 4.0 | 4.0 | 4.0 | 4.0 | 4.0 |
| Polyvinylpyrrolidone (Kollidon 30) | 10.0 | 10.0 | 10.0 | 10.0 | 10.0 | 10.0 | 10.0 |
| Butylated Hydroxyanisole | 1.0 | 1.0 | 1.0 | 1.0 | 1.0 | 1.0 | 1.0 |
| Norethindrone Acetate | 3.0 | 3.0 | 3.0 | 3.0 | 3.0 | 3.0 | 3.0 |
| Estradiol | 0.1 | 0.2 | 0.4 | 0.5 | 0.6 | 0.8 | 1.0 |
| | 100.0 | 100.0 | 100.0 | 100.0 | 100.0 | 100.0 | 100.0 |

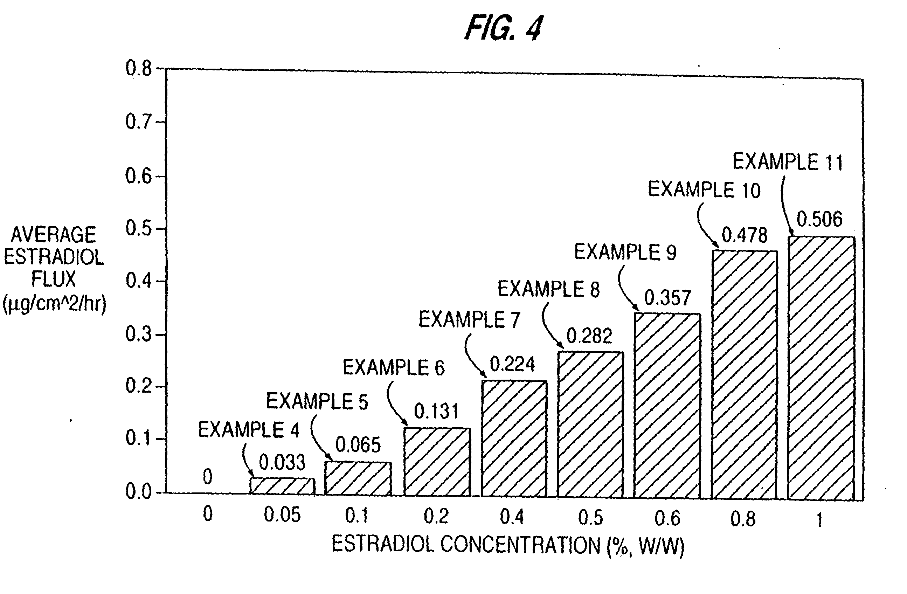

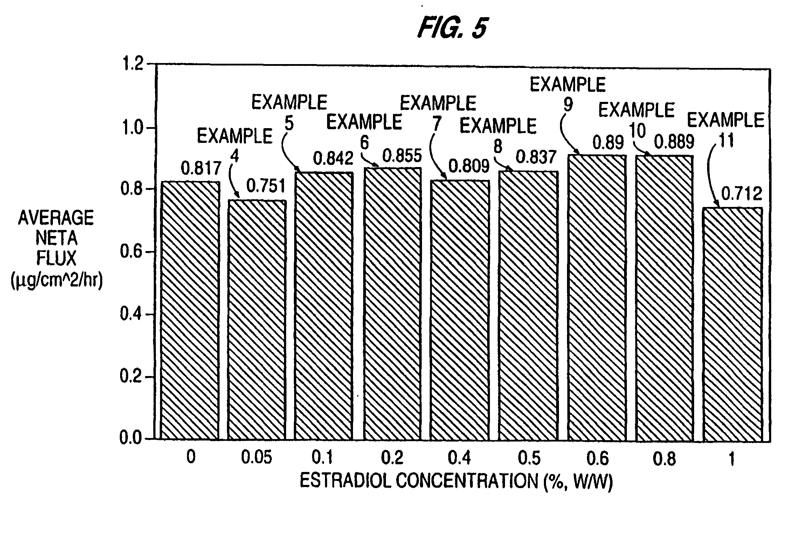

Estradiol flux through human epidermis in vitro from systems of Examples 4 through 11 (with 0.05% to 1.0% estradiol) are presented in Figure 4. This graph shows how a wide range in estradiol flux was achieved by the formulas of this invention by varying the estradiol concentration. Norethindrone acetate flux was not affected by estradiol concentration, and remained constant at about 0.8 µg/hr; see Figure 5.

### COMPARATIVE EXAMPLE 12

An estradiol/norethindrone acetate-polymer mixture was prepared by combining 0.2 parts of estradiol, 3.0 parts of norethindrone acetate, 4.0 parts of dipropylene glycol, 6.0 parts of oleic acid, 60.0 parts of toluene, 0.0 parts of polyvinylpyrrolidone (Kollidon 30), 1.0 parts of butylated hydroxyanisole, and 64.52 parts of polyacrylate adhesive (GMS 737) in an appropriate container, and mixing well until the mixture was completely homogeneous. Then 93.0 parts of polysiloxane adhesive (BIO-PSA Q7-4503) were added, and the blend was thoroughly mixed. The resulting composition has the ingredient concentrations on a "dry" basis, that is, after removal of volatile process solvents in ovens, given below.

| COMPONENT | PERCENT BY WEIGHT |
|---|---|
| Polysiloxane Adhesive (BIO-PSA Q7-4503) | 65.8 |
| Polyacrylate Adhesive (GMS 737) | 20.0 |
| Oleic Acid | 6.0 |
| Dipropylene Glycol | 4.0 |
| Polyvinylpyrrolidone (Kollidon 30) | 0.0 |
| Butylated Hydroxyanisole | 1.0 |
| Norethindrone Acetate | 3.0 |
| Estradiol | 0.2 |
| | $\overline{\text{100.0}}$ |

In the following examples, the method of Example 12 was used with the appropriate amounts of starting materials to yield compositions having the following ingredient concentrations.

| | **EXAMPLE 13** | **EXAMPLE 14** |
|---|---|---|
| COMPONENT | PERCENT BY WEIGHT | |
| Polysiloxane Adhesive (BIO-PSA Q7-4503) | 63.3 | 60.8 |
| Polyacrylate Adhesive (GMS 737) | 20.0 | 20.0 |
| Oleic Acid | 6.0 | 6.0 |
| Dipropylene Glycol | 4.0 | 4.0 |
| Polyvinylpyrrolidone (Kollidon 30) | 2.5 | 5.0 |
| Butylated Hydroxyanisole | 1.0 | 1.0 |
| Norethindrone Acetate | 3.0 | 3.0 |
| Estradiol | 0.2 | 0.2 |
| | 100.0 | 100.0 |

### EXAMPLE 15

| COMPONENT | PERCENT BY WEIGHT |
|---|---|
| Polysiloxane Adhesive (BIO-PSA X7-4603) | 71.3 |
| Polyacrylate Adhesive (GMS 737) | 5.0 |
| Dipropylene Glycol | 4.0 |
| Oleic Acid | 6.0 |
| Polyvinylpyrrolidone (Kollidon 30) | 10.0 |
| Norethindrone Acetate | 3.0 |
| Estradiol | 0.7 |
| | $\overline{\text{100.0}}$ |

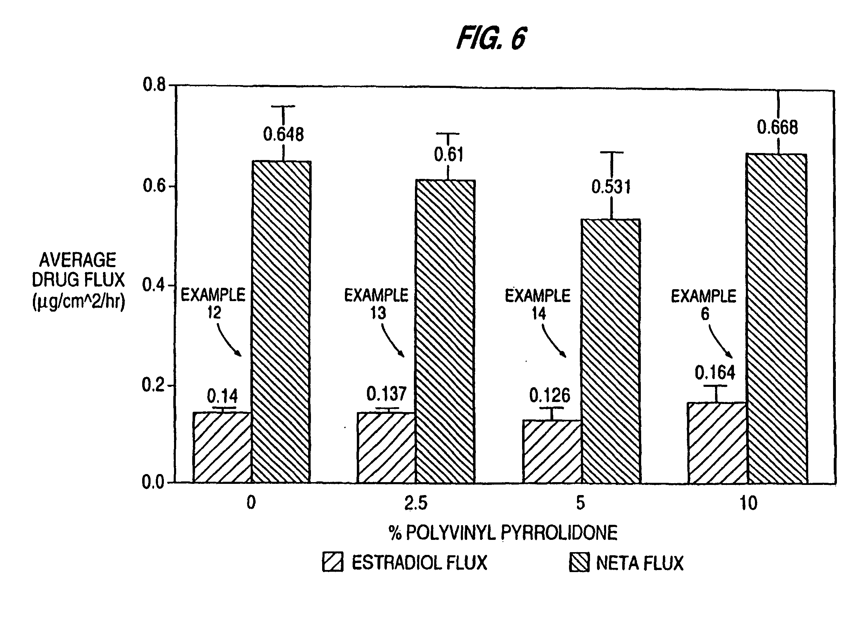

Figure 6 shows how systems with varying levels of polyvinylpyrrolidone (0 to 10%) had essentially the same drug (estradiol and norethindrone acetate) flux; Examples 6, 12-14. However, polyvinylpyrrolidone was found to have an effect on drug recrystallization for these systems. That is, the incidence of crystal formation was reduced as the polyvinylpyrrolidone concentration was increased; see Table III.

**Table III.**

| Effects of polyvinylpyrrolidone on crystal formation. | | |
|---|---|---|
| Formula | % polyvinylpyrrolidone | # of crystals in patch* |
| Example 12 | 0.0 | 60 ± 4 |
| Example 13 | 2.5 | 56 ± 8 |
| Example 14 | 5.0 | 20 ± 4 |
| Example 6 | 10.0 | 0 |

| | | |
|---|---|---|
| * number of visible crystals in a 14.4 cm² patch; average and sd of five patches each. | | |

### EXAMPLE 16

An isosorbide dinitrate-polymer mixture is prepared by combining 20.0 parts of isosorbide dinitrate, 4.0 parts of dipropylene glycol, 4.0 parts of oleic acid, 10.0 parts of polyvinylpyrrolidone (Kollidon 30) and 67.0 parts of polyacrylate adhesive (Duro-Tak 80-1196) in an appropriate container, and mixing well until the mixture is completely homogeneous. In this example, the isosorbide dinitrate is added as a solution in toluene mixed together with the polyacrylate adhesive. Then 53.0 parts of polysiloxane adhesive (BIO-PSA Q7-4503) are added, and the blend is thoroughly mixed. The resulting composition has the ingredient concentrations on a "dry" basis, that is, after removal of volatile process solvents in ovens, given below.

| COMPONENT | PERCENT BY WEIGHT |
|---|---|
| Polysiloxane Adhesive (BIO-PSA Q7-4503) | 32.0 |
| Polyacrylate Adhesive (Duro-Tak 80-1196) | 30.0 |
| Polyvinylpyrrolidone (Kollidon 30) | 10.0 |
| Dipropylene Glycol | 4.0 |
| Oleic Acid | 4.0 |
| Isosorbide Dinitrate | 20.0 |
| | $\overline{\text{100.0}}$ |

### EXAMPLE 17

A norethindrone acetate-polymer mixture is prepared by combining 3.0 parts of norethindrone acetate, 4.0 parts of dipropylene glycol, 6.0 parts of oleic acid, 60.0 parts of toluene, 10.0 parts of polyvinylpyrrolidone (Kollidon VA 64), 1.0 parts of butylated hydroxyanisole, and 64.52 parts of polyacrylate adhesive (GMS 737) in an appropriate container, and mixing well until the mixture is completely homogeneous. Then 95.00 parts of polysiloxane adhesive (BIO-PSA X7-4603) are added, and the blend is thoroughly mixed. The resulting composition has the ingredient concentrations on a "dry" basis, that is, after removal of volatile process solvents, given below. Although the following embodiments contain 2.0 to 3.0% norethindrone acetate, a preferred range is from about 1 to about 12% by weight.

| COMPONENT | PERCENT BY WEIGHT |
|---|---|
| Polysiloxane Adhesive (BIO-PSA X7-4603) | 57.0 |
| Polyacrylate Adhesive (GMS 737) | 20.0 |
| Dipropylene Glycol | 4.0 |
| Oleic Acid | 6.0 |
| Polyvinylpyrrolidone (Kollidon VA 64) | 10.0 |
| Norethindrone Acetate | 3.0 |
| | $\overline{\text{100.0}}$ |

In the following examples, the method of Example 17 is used with the appropriate amounts of starting materials to yield compositions having the following ingredient concentrations.

| Examples | 18 | 20 | 21 | C 22 | 23 | 24 | 25 |
|---|---|---|---|---|---|---|---|
| COMPONENT | PERCENT BY WEIGHT | | | | | | |
| Polysiloxane Adhesive (BIO-PSA X7-4603) | 54.7 | 26.2 | 12.0 | 0.0 | 0.0 | 0.0 | 0.0 |
| Polysiloxane Adhesive (BIO-PSA Q7-4502) | 0.0 | 0.0 | 0.0 | 5.0 | 0.0 | 0.0 | 0.0 |
| Polysiloxane Adhesive (BIO-PSA X7-4301 | 0.0 | 0.0 | 0.0 | 0.0 | 52.0 | 0.0 | 0.0 |
| Polysiloxane Adhesive (BIO-PSA Q7-4501) | 0.0 | 0.0 | 0.0 | 0.0 | 0.0 | 65.0 | 65.0 |
| Polyacrylate Adhesive (GMS 737) | 23.3 | 0.0 | 0.0 | 0.0 | 0.0 | 0.0 | 0.0 |
| Polyacrylate Adhesive (Duro-Tak 80-1196) | 0.0 | 45.3 | 0.0 | 0.0 | 20.0 | 0.0 | 0.0 |
| Polyacrylate Adhesive (Duro-Tak 87-2852) | 0.0 | 0.0 | 0.0 | 70.0 | 0.0 | 15.0 | 15.0 |
| Polyacrylate Adhesive (Gelva 788) | 0.00 | 0.00 | 60.0 | 0.0 | 0.0 | 0.0 | 0.0 |
| Oleic Acid | 5.8 | 2.0 | 2.0 | 5.0 | 8.0 | 0.0 | 0.0 |
| Citric Acid | 0.0 | 0.0 | 0.0 | 5.0 | 0.0 | 0.0 | 0.0 |
| Dipropylene Glycol | 3.9 | 4.0 | 6.0 | 0.0 | 5.0 | 0.0 | 0.0 |
| Polyoxyethylene (4) Lauryl Ether (Brij 30) | 0.0 | 6.0 | 5.0 | 0.0 | 0.0 | 0.0 | 0.0 |
| Polyvinylpyrrolidone (Kollidon 30) | 9.6 | 9.5 | 0.0 | 0.0 | 10.0 | 0.0 | 0.0 |
| Polyvinylpyrrolidone (Kollidon 17PF) | 0.0 | 0.0 | 5.0 | 5.0 | 0.0 | 0.0 | 0.0 |
| Polyvinylpyrrolidone (Kollidon 90) | 0.0 | 0.0 | 0.0 | 0.0 | 0.0 | 5.0 | 5.0 |
| Drug (Norethindrone Acetate) | 2.7 | 0.0 | 0.0 | 0.0 | 0.0 | 0.0 | 0.0 |
| Alprazolam | 0.0 | 7.0 | 0.0 | 0.0 | 0.0 | 0.0 | 0.0 |
| Albuterol | 0.0 | 0.0 | 10.0 | 0.0 | 0.0 | 0.0 | 0.0 |
| δ-Aminolevulinic Acid | 0.0 | 0.0 | 0.0 | 10.0 | 0.0 | 0.0 | 0.0 |
| Fentanyl | 0.0 | 0.0 | 0.0 | 0.0 | 5.0 | 0.0 | 0.0 |
| Nicotine | 0.0 | 0.0 | 0.0 | 0.0 | 0.0 | 15.0 | 0.0 |
| Selegiline | 0.0 | 0.0 | 0.0 | 0.0 | 0.0 | 0.0 | 15.0 |
| | 100.0 | 100.0 | 100.0 | 100.0 | 100.0 | 100.0 | 100.0 |

### COMPARATIVE EXAMPLE 26

| COMPONENT | PERCENT BY WEIGHT |
|---|---|
| Polysiloxane Adhesive (BIO-PSA Q7-4503) | 5.0 |
| Polyacrylate Adhesive (Gelva 737) | 60.0 |
| Polyvinylpyrrolidone (Kollidon 90) | 5.0 |
| Ketoprofen | 30.0 |
| | $\overline{\text{100.0}}$ |

### COMPARATIVE EXAMPLE 27

An estradiol-polymer mixture was prepared by combining 2.0 parts of estradiol, 4.0 parts of dipropylene glycol, 4.0 parts of oleic acid, 3.0 parts of lecithin and 5.0 parts of polyvinylpyrrolidone (Kollidon 17PF) in an appropriate container, and mixing well until the mixture was completely homogeneous. In this example, the estradiol is added (as a solution in toluene mixed together) with 67.0 parts polyisobutylene (Vistanex LM-LS-LC). Then 124.0 parts of polysiloxane adhesive (BIO-PSA X7-4301) were added, and the blend was thoroughly mixed. The resulting composition has the ingredient concentrations on a "dry" basis, that is, after removal of volatile process solvents, given below.

| COMPONENT | PERCENT BY WEIGHT |
|---|---|
| Polysiloxane Adhesive (BIO-PSA X7-4301) | 62.0 |
| Polyisobutylene (Vistanex LM-LS-LC) | 20.0 |
| Dipropylene Glycol | 4.0 |
| Oleic Acid | 4.0 |
| Polyvinylpyrrolidone (Kollidon 17PF) | 5.0 |
| Lecithin | 3.0 |
| Estradiol | 2.0 |
| | $\overline{\text{100.0}}$ |

In the following examples, the method of Example 27 is used with the appropriate amounts of starting materials to yield compositions having the following ingredient concentrations:

### EXAMPLE 28

| COMPONENT | PERCENT BY WEIGHT |
|---|---|
| Polyacrylate Adhesive (GMS 737) | 55.0 |
| Polyisobutylene (Vistanex LM-LS-LC) | 20.0 |
| Dipropylene Glycol | 5.0 |
| Oleic Acid | 8.0 |
| Polyvinylpyrrolidone (Kollidon 30) | 10.0 |
| Haloperidol | 2.0 |
| | $\overline{\text{100.0}}$ |

### COMPARATIVE EXAMPLE 29

| COMPONENT | PERCENT BY WEIGHT |
|---|---|
| Polyacrylate Adhesive (Duro-Tak 80-1196) | 69.0 |
| Polyvinylpyrrolidone (Kollidon 30) | 10.0 |
| Butylene Glycol | 5.0 |
| Oleic Acid | 8.0 |
| Tocopherol Acetate (Vitamin E Acetate) | 3.0 |
| Fentanyl | 5.0 |
| | $\overline{\text{100.0}}$ |

### EXAMPLE 30

An estradiol-polymer mixture is prepared by combining 1.6 parts of estradiol, 6.0 parts of dipropylene glycol, 8.0 parts of oleic acid, 4.8 parts of polyvinylpyrrolidone (Kollidon 30), 50.0 parts of polyacrylate adhesive (GMS 1430), 17.0 parts of polysiloxane A adhesive (BIO-PSA X7-4603) and 82.0 parts of polysiloxane B adhesive (BIO-PSA Q7-4503) in an appropriate container, and mixing well until the mixture is completely homogeneous. The resulting composition has the ingredient concentrations on a "dry" basis, that is, after removal of volatile process solvents, given below.

| COMPONENT | PERCENT BY WEIGHT |
|---|---|
| Polysiloxane A Adhesive (BIO-PSA X7-4603) | 10.0 |
| Polysiloxane B Adhesive (BIO-PSA Q7-4503) | 49.5 |
| Polyacrylate Adhesive (GMS 1430) | 20.1 |
| Dipropylene Glycol | 6.0 |
| Oleic Acid | 8.0 |
| Polyvinylpyrrolidone (Kollidon 30) | 4.8 |
| Estradiol | 1.6 |
| | $\overline{\text{100.0}}$ |

In the following example, the method of Example 30 is used with the appropriate amounts of starting materials to yield compositions having the following ingredient concentrations.

### EXAMPLE 31

| COMPONENT | PERCENT BY WEIGHT |
|---|---|
| Polysiloxane A Adhesive (BIO-PSA Q7-4503) | 5.0 |
| Polysiloxane B Adhesive (BIO-PSA X7-4603) | 71.6 |
| Polyacrylate Adhesive (GMS 737) | 5.0 |
| Oleamide | 6.0 |
| Dipropylene Glycol | 2.0 |
| Polyvinylpyrrolidone (Kollidon 30) | 5.0 |
| Polyoxyethylene (2) Oleyl Ether (Brij 93) | 2.0 |
| Norethindrone Acetate | 3.0 |
| Estradiol | 0.4 |
| | $\overline{\text{100.0}}$ |

### EXAMPLE 32

| COMPONENT | PERCENT BY WEIGHT |
|---|---|
| Polysiloxane A Adhesive (BIO-PSA Q7-4503) | 5.0 |
| Polysiloxane B Adhesive (BIO-PSA X7-4603) | 71.6 |
| Polyacrylate Adhesive (GMS 737) | 5.0 |
| Oleamide | 6.0 |
| Dipropylene Glycol | 4.0 |
| Polyvinylpyrrolidone (Kollidon 30) | 5.0 |
| Norethindrone Acetate | 3.0 |
| Estradiol | 0.4 |
| | $\overline{\text{100.0}}$ |

### EXAMPLE 33

| COMPONENT | PERCENT BY WEIGHT |
|---|---|
| Polysiloxane Adhesive (BIO-PSA X7-4603) | 71.2 |
| Polyacrylate Adhesive (GMS 737) | 5.0 |
| Oleyl Alcohol | 6.0 |
| Polyoxyethylene (2) Oleyl Ether (Brij 93) | 4.0 |
| Polyvinylpyrrolidone (Kollidon VA 64) | 10.0 |
| Norethindrone Acetate | 3.0 |
| Estradiol | 0.8 |
| | $\overline{\text{100.0}}$ |

## Claims

1. A pressure-sensitive adhesive composition suitable for use in a transdermal drug delivery system, which composition comprises a blend of:
(a) a rubber adhesive, wherein die rubber adhesive is present in an amount from 9% to 94% by weight of the total composition;
(b) a polyacrylate in an amount from 2% to 85% by weight of the total composition, wherein the ratio of the polyacrylate to the rubber adhesive is from 2:98 to 96:4;
(c) a therapeutically effective amount of one drug or a mixture of two or more drugs for transdermal drug delivery, wherein the drug is present in an amount of 0.1% to 50% of the total composition; and
(d) a soluble polyvinylpyrrolidone, wherein the soluble polyvinylpyrrolidone is present in an amount of 1% to 20% of the total composition,
wherein ratio of the drug to the soluble polyvinylpyrrolidone is from 1:10 to 10:1, and
wherein the amount of soluble polyvinylpyrrolidone is sufficient to solubilize all of the drug which is present in an amount that would exceed its solubility in a composition that contains a rubber adhesive and a polyacrylate, but lacks soluble polyvinylpyrrolidone.

2. A composition of claim 1, comprising 9% to 94% polysiloxane as the rubber adhesive component, 2% to 85% polyacrylate, 1% to 15% polyvinylpyrrolidone, 0 to 20% co-solvent, 0% to 15% enhancer and 0.3% to 30% drug, based on percent by weight of the total composition.

3. A composition according to claim 1 or 2, wherein the polyvinylpyrrolidone has a molecular weight from 7,000 to 54,000.

4. A composition according to any one of claims 1 to 3, wherein the druo is selected from die group consisting of steroids, β₂-adrenergic agonists and blockers, cardioactive agents, cholinergic agonists, tranquilizers, anesthetics, analgesics, anti-neopalstics, central nervous system acting drugs, vasodilators, antiparkinsonian drugs, antipsychotic drugs and non-steroidal anti-inflammatory drugs.

5. A composition according to claim 4, comprising at least two drugs.

6. A composition according to claim 4 wherein the drug is selected from the group consisting of conjugated estrogenic hormones, esterified estrogens, estropipate, 17β-estradiol, equilin, mestranol, estrone, estriol, ethinyl estradiol, diethylstilbestrol progesterone, 19-norprogesterone, norethindrone, norethindrone acetate, melengestrol, chlormadinone, ethisterone, medroxyprogesterone acetate, hydroxyprogesterone caproate, ethynodiol diacetate, norethynodrel, 17α-hydroxyprogesterone, dydrogesterone, dimethisterone, ethynylestrenol, norgestrel, demegestone, promegestone, megestrol acetate, metaproterenol. terbutaline, albuterol, carbuterol, rimiterol, fenoterol, soterenol, nitroglycerin, isosorbide dimitrate, isosorbide mononitrate, quinidine sulfate, procainamide, bendroflumethiazide, benzthiazide, chlorothiazide, nifedipine, nicardipine, verapamil, diltiazem, timolol, propanolol, captopril, clonidine, prazosin, choline, acetylcholine, methachoine, carbachol, bethanechol, pilocarpine, muscarine, arecoline, alprazolam, chlordiazepoxide, clorazeptate, halazepam, oxazepam, prazepam, clonazepam, flurazepam, triazolam, lorazepam, diazepam, thiopropazate, chlorpromazine, triflupromazine, mesoridazine, piperacetazine, thioridazine, acetophenazine, fluphenazine, perphenazine, trifluoperazine, chlorprothixene, thiothixene, haloperidol, bromperidol, loxapine, molidone, lidocaine, tetracaine, dyclonine, dibucaine, procaine, mepivacaine, bupivacaine, etidocaine, prilocaine, benzocaine, fentanyl, buprenorphine, codeine, nicotine, papaverine, butorphanol, hydromorphone, oxymorphone, mecamylamine, diclofenac, fenoprofen, flurbiprofen, ibuprofen, ketoprofen, naproxen, piroxicam, δ-amino-levulinic acid, deprenyl, selegiline, buspirone and lisuride.

7. A composition according to claim 3, comprising 5 to 10% of polyvinylpyrrolidone.

8. A composition according to claim 5, comprising a mixture of a progestational agent and an estrogen.

9. The composition of claim 8, comprising 71.3% polysiloxane, 5% polyacrylate, 10% polyvinylpyrrolidone, 3% norethindrone acetate and 0.7% estradiol.

10. The composition of claim 6, comprising 71.2% polysiloxane, 5% polyacrylate, 10% polyvinylpyrrolidone, and 3% norethindrone acetate.

11. The composition of claim 6, comprising 26.2% polysiloxane, 45.3% polyacrylate, 9.5 % polyvinylpyrrolidone, 7% alprazolam and 10% enhancer.

12. The composition of claim 6, comprising 12% polysiloxane, 60% polyacrylate, 5% polyvinylpyrrolidone, and 10% albuterol.

13. The composition of claim 6, comprising 5% polysiloxane, 70% polyacrylate, about 5% polyvinylpyrrolidone, and 10% δ-aminolevulinic acid.

14. The composition of claim 6, comprising 52% polysiloxane, 20% polyacrylate, 10% polyvinylpyrrolidone, and 5% fentanyl.

15. The composition of claim 6, comprising 65% polysiloxane, 15% polyacrylate, 5% polyvinylpyrrolidone, and 15% nicotine.

16. The composition of claim 6, comprising 65% polysiloxane, 15% polyacrylate, 5% polyvinylpyrrolidone, and 15% selegiline.

17. The composition of claim 6, comprising 5% polysiloxane, 60% polyacrylate, 5% polyvinylpyrrolidone, and 30% ketoprofen.

18. The composition of claim 6, wherein the drug is 17β-estradiol.

19. A composition according to claim 1, wherein the composition has a glass transition temperature of from about -70°C to 0°C.

20. A composition according to claim 1, further comprising an enhancer.

21. A composition according to claim 1, further comprising a clay.

22. A composition according to claim 21, wherein the clay is bentonite.

23. A composition according to claim 1, wherein the drug is selected from the group consisting of fluoxetine, sertaline and paroxetine.

24. A composition according to claim 1, wherein die drug includes clonidine.

25. A composition according to claim 1, wherein the drug is selected from the group consisting of bromocriptine, lisuride, and pergolide.

26. A composition according to claim 1, wherein the drug is selected from the group consisting of norgestimate, norgestrel, ethynodiol diacetate, and desogestrel.

27. A composition according to claim 1, wherein the drug is selected from the group consisting of ipsapirone and buspirone.

28. A composition according to claim 1, wherein the drug comprises alprazolam.

29. A composition according to claim 1, wherein the drug comprises clonazepam.

30. A composition according to claim 1, wherein the drug comprises clonidine.

31. The composition of claim 1, wherein the drug comprises fludrocortisone acetate.

32. The composition of claim 1, wherein the drug comprises ketoprofen.

33. The composition of claim 1, wherein the drug comprises estradiol.

34. A transdermal drug delivery system comprising a sheet of defined geometric shape comprising a composition as claimed in any one of the preceding claims.

35. A system according to claim 34 which is in the form of an individual dosage unit.

36. A system according to claim 34, further comprising a backing material superimposed on one surface of the composition, the backing material being substantially impermeable to the drug, and a release liner superimposed on the surface of the composition opposite the backing.

37. A method of preparing a pressure-sensitive adhesive composition for transdermal drug delivery, which comprises blending of:
(a) a rubber adhesive in an amount of from 9% to 94% by weight of the total composition;
(b) a polyacrylate in an amount of from 2% to 85% by weight of the total composition wherein the ratio of the polyacrylate to the rubber adhesive is from 2:98 to 96:4;
(c) a therapeutically effective amount of one drug or a mixture of two or more drugs for transdermal drug delivery, wherein the drug is present in an amount of 0.1% to 50% of the total composition; and
(d) a soluble polyvinylpyrrolidone, wherein the soluble polyvinylpyrrolidone is present in an amount of 1% to 20% of the total composition,
wherein the ratio of the drug to the soluble polyvinylpyrrolidone is from 1:10 to 10:1, and
wherein the amount of soluble polyvinylpyrrolidone is sufficient to solubilize all of the drug which is present in an amount that would exceed its solubility in a composition that contains a rubber adhesive : and a polyacrylate, but lacks soluble polyvinylpyrrolidone.

38. A composition according to any one of claims I to 3, wherein the drug is selected from the group consisting of enalapril, enalaprilat, fluoxetine, niflumic acid, piroxicam and sertraline.

## Patentansprüche

1. Haftkleberzusammensetzung, die zur Verwendung in einem transdermalen Wirkstoffabgabesystem geeignet ist, wobei die Zusammensetzung ein Gemisch umfasst aus:
(a) einem Kautschukkleber, wobei der Kautschukkleber in einer Menge von 9 bis 94 Gew.-% der Gesamtzusammensetzung vorhanden ist;
(b) einem Polyacrylat in einer Menge von 2 bis 85 Gew.-% der Gesamtzusammensetzung, wobei das Verhältnis des Polyacrylats zu dem Kautschukkleber 2:98 bis 96:4 beträgt;
(c) einer therapeutisch wirksamen Menge eines Wirkstoffs oder eines Gemischs von zwei oder mehr Wirkstoffen für die transdermale Wirkstoffabgabe, wobei der Wirkstoff in einer Menge von 0,1 bis 50 Gew.-% der Gesamtzusammensetzung vorhanden ist; und
(d) eines löslichen Polyvinylpyrrolidons, wobei das lösliche Polyvinylpyrrolidon in einer Menge von 1 bis 20 Gew.-% der Gesamtzusammensetzung vorhanden ist;
wobei das Verhältnis des Wirkstoffs zu dem löslichen Polyvinylpyrrolidon 1:10 bis 10:1 beträgt; und
wobei die Menge des löslichen Polyvinylpyrrolidons ausreichend ist, um den gesamten Wirkstoff in Lösung zu bringen, welcher in einer Menge vorhanden ist, die seine Löslichkeit in einer Zusammensetzung, die einen Kautschukkleber und ein Polyacrylat, aber kein lösliches Polyvinylpyrrolidon enthält, überschreiten würde.

2. Zusammensetzung gemäß Anspruch 1 mit 9 bis 94 Gew.-% Polysiloxan als Kautschukkleberkomponente, 2 bis 85 Gew.-% Polyacrylat, 1 bis 15 Gew.-% Polyvinylpyrrolidon, 0 bis 20 Gew.-% Cosolvens, 0 bis 15 Gew.-% Verstärker und 0,3 bis 30 Gew.-% Wirkstoff, bezogen auf das Gewicht der Gesamtzusammensetzung.

3. Zusammensetzung gemäß Anspruch 1 oder 2, wobei das Polyvinylpyrrolidon ein Molekulargewicht von 7000 bis 54 000 hat.

4. Zusammensetzung gemäß einem der Ansprüche 1 bis 3, wobei der Wirkstoff aus der Gruppe ausgewählt ist, die aus Steroiden, β₂-adrenergischen Agonisten und Blockern, herzaktiven Wirkstoffen, cholinergischen Agonisten, Tranquilizern, Anästhetika, Analgetika, Aritineoplastika, auf das Zentralnervensystem wirkenden Medikamenten, Vasodilatoren, Parkinson-Medikamenten, Antipsychotika sowie nichtsteroidalen entzündungshemmenden Medikamenten besteht.

5. Zusammensetzung gemäß Anspruch 4, die wenigstens zwei Wirkstoffe umfasst.

6. Zusammensetzung gemäß Anspruch 4, wobei der Wirkstoff aus der Gruppe ausgewählt ist, die aus konjugierten estrogenischen Hormonen, veresterten Estrogenen, Estropipat, 17β-Estradiol, Equilin, Mestranol, Estron, Estriol, Ethinylestradiol, Diethylstilbestrol, Progesteron, 19-Norprogesteron, Norethindron, Norethindronacetat, Melengestrol, Chlormadinon, Ethisteron, Medroxyprogesteronacetat, Hydroxyprogesteroncaproat, Ethinodioldiacetat, Norethinodrel, 17α-Hydroxyprogesteron, Dydrogesteron, Dimethisteron, Ethinylestrenol, Norgestrel, Demegeston, Promegeston, Megestrolacetat, Metaproterenol, Terbutalin, Albuterol, Carbuterol, Rimiterol, Fenoterol, Soterenol, Nitroglycerin, Isosorbiddinitrat, Isosorbidmononitrat, Chinidinsulfat, Procainamid, Bendroflumethiazid, Benzthiazid, Chlorothiazid, Nifedipin, Nicardipin, Verapamil, Diltiazem, Timolol, Propanolol, Captopril, Clonidin, Prazosin, Cholin, Acetylcholin, Methacholin, Carbachol, Bethanechol, Pilocarpin, Muscarin, Arecolin, Alprazolam, Chlordiazepoxid, Chlorazeptat, Halazepam, Oxazepam, Prazepam, Clonazepam, Flurazepam, Triazolam, Lorazepam, Diazepam, Thiopropazat, Chlorpromazin, Triflupromazin, Mesoridazin, Piperacetazin, Thioridazin, Acetophenazin, Fluphenazin, Perphenazin, Trifluoperazin, Chlorprothixen, Thiothixen, Haloperidol, Bromperidol, Loxapin, Molidon, Lidocain, Tetracain, Dyclonin, Dibucain, Cocain, Procain, Mepivacain, Bupivacain, Etidocain, Prilocain, Benzocain, Fentanyl, Buprenorphin, Codein, Nicotin, Papaverin, Butorphanol, Hydromorphon, Oxymorphon, Mecamylamin, Diclofenac, Ferioprofen, Flurbiprofen, Ibuprofen, Ketoprofen, Naproxen, Piroxicam, δ-Aminolävulinsäure, Deprenyl, Selegilin, Buspiron und Lisurid besteht.

7. Zusammensetzung gemäß Anspruch 3, die 5 bis 10% Polyvinylpyrrolidon umfasst.

8. Zusammensetzung gemäß Anspruch 5, die ein Gemisch aus einem Gestagen und einem Estrogen umfasst.

9. Zusammensetzung gemäß Anspruch 8, die 71,3% Polysiloxan, 5% Polyacrylat, 10% Polyvinylpyrrolidon, 3% Norethindronacetat und 0,7% Estradiol umfasst.

10. Zusammensetzung gemäß Anspruch 6, die 71,2% Polysiloxan, 5% Polyacrylat, 10% Polyvinylpyrrolidon und 3% Norethindronacetat umfasst.

11. Zusammensetzung gemäß Anspruch 6, die 26,2% Polysiloxan, 45,3% Polyacrylat, 9,5% Polyvinylpyrrolidon, 7% Alprazolam und 10% Verstärker umfasst.

12. Zusammensetzung gemäß Anspruch 6, die 12% Polysiloxan, 60% Polyacrylat, 5% Polyvinylpyrrolidon und 10% Albuterol umfasst.

13. Zusammensetzung gemäß Anspruch 6, die 5% Polysiloxan, 70% Polyacrylat, etwa 5% Polyvinylpyrrolidon und 10% δ-Aminolävulinsäure umfasst.

14. Zusammensetzung gemäß Anspruch 6, die 52% Polysiloxan, 20% Polyacrylat, 10% Polyvinylpyrrolidon und 5% Fentanyl umfasst.

15. Zusammensetzung gemäß Anspruch 6, die 65% Polysiloxan, 15% Polyacrylat, 5% Polyvinylpyrrolidon und 15% Nicotin umfasst.

16. Zusammensetzung gemäß Anspruch 6, die 65% Polysiloxan, 15% Polyacrylat, 5% Polyvinylpyrrolidon und 15% Selegilin umfasst.

17. Zusammensetzung gemäß Anspruch 6, die 5% Polysiloxan, 60% Polyacrylat, 5% Polyvinylpyrrolidon und 30% Ketoprofen umfasst.

18. Zusammensetzung gemäß Anspruch 6, wobei es sich bei dem Wirkstoff um 17β-Estradiol handelt.

19. Zusammensetzung gemäß Anspruch 1, wobei die Zusammensetzung eine Glasübergangstemperatur von etwa -70 °C bis 0 °C hat.

20. Zusammensetzung gemäß Anspruch 1, die weiterhin einen Verstärker umfasst.

21. Zusammensetzung gemäß Anspruch 1, die weiterhin einen Ton umfasst.

22. Zusammensetzung gemäß Anspruch 21, wobei es sich bei dem Ton um Bentonit handelt.

23. Zusammensetzung gemäß Anspruch 1, wobei der Wirkstoff aus der Gruppe ausgewählt ist, die aus Fluoxetin, Sertalin und Paroxetin besteht.

24. Zusammensetzung gemäß Anspruch 1, wobei der Wirkstoff Clonidin umfasst.

25. Zusammensetzung gemäß Anspruch 1, wobei der Wirkstoff aus der Gruppe ausgewählt ist, die aus Bromocriptin, Lisurid und Pergolid besteht.

26. Zusammensetzung gemäß Anspruch 1, wobei der Wirkstoff aus der Gruppe ausgewählt ist, die aus Norgestimat, Norgestrel, Ethinodioldiacetat und Desogestrel besteht.

27. Zusammensetzung gemäß Anspruch 1, wobei der Wirkstoff aus der Gruppe ausgewählt ist, die aus Ipsapiron und Buspiron besteht.

28. Zusammensetzung gemäß Anspruch 1, wobei der Wirkstoff Alprazolam umfasst.

29. Zusammensetzung gemäß Anspruch 1, wobei der Wirkstoff Clonazepam umfasst.

30. Zusammensetzung gemäß Anspruch 1, wobei der Wirkstoff Clonidin umfasst.

31. Zusammensetzung gemäß Anspruch 1, wobei der Wirkstoff Fluodrocortisonacetat umfasst.

32. Zusammensetzung gemäß Anspruch 1, wobei der Wirkstoff Ketoprofen umfasst.

33. Zusammensetzung gemäß Anspruch 1, wobei der Wirkstoff Estradiol umfasst.

34. Transdermales Wirkstoffabgabesystem, das ein Fiächengebilde mit definierter geometrischer Form umfasst, welches eine Zusammensetzung gemäß einem der vorstehenden Ansprüche umfasst.

35. System gemäß Anspruch 34, das in Form einer einzelnen Dosierungseinheit vorliegt.

36. System gemäß Anspruch 34, das weiterhin ein Trägermaterial umfasst, das über eine Oberfläche der Zusammensetzung gelegt ist, wobei das Trägermaterial für den Wirkstoff im Wesentlichen undurchlässig ist, und eine Trennfolie über die Oberfläche gegenüber dem Träger gelegt ist.

37. Verfahren zur Herstellung einer Haftkleberzusammensetzung für die transdermale Wirkstoffabgabe, umfassend das Mischen von:
(a) einem Kautschukkleber in einer Menge von 9 bis 94 Gew.-% der Gesamtzusammensetzung;
(b) einem Polyacrylat in einer Menge von 2 bis 85 Gew.-% der Gesamtzusammensetzung, wobei das Verhältnis des Polyacrylats zu dem Kautschukkleber 2:98 bis 96:4 beträgt;
(c) einer therapeutisch wirksamen Menge eines Wirkstoffs oder eines Gemischs von zwei oder mehr Wirkstoffen für die transdermale Wirkstoffabgabe, wobei der Wirkstoff in einer Menge von 0,1 bis 50 Gew.-% der Gesamtzusammensetzung vorhanden ist; und
(d) eines löslichen Polyvinylpyrrolidons, wobei das lösliche Polyvinylpyrrolidon in einer Menge von 1 bis 20 Gew.-% der Gesamtzusammensetzung vorhanden ist;
wobei das Verhältnis des Wirkstoffs zu dem löslichen Polyvinylpyrrolidon 1:10 bis 10:1 beträgt; und
wobei die Menge des löslichen Polyvinylpyrrolidons ausreichend ist, um den gesamten Wirkstoff in Lösung zu bringen, welcher in einer Menge vorhanden ist, die seine Löslichkeit in einer Zusammensetzung, die einen Kautschukkleber und ein Polyacrylat, aber kein lösliches Polyvinylpyrrolidon enthält, überschreiten würde.

38. Zusammensetzung gemäß einem der Ansprüche 1 bis 3, wobei der Wirkstoff aus der Gruppe ausgewählt ist, die aus Enalapril, Enalaprilat, Fluoxetin, Nifluminsäure, Piroxicam und Sertralin besteht.

## Revendications

1. Une composition adhésive sensible à la pression utilisable dans un système d'administration transdermique de médicament, laquelle composition comprend un mélange de :
(a) un adhésif caoutchoutique, l'adhésif caoutchoutique étant présent en une quantité de 9 % à 94 % en poids de la composition totale ;
(b) un polyacrylate en une quantité de 2 % à 85 % en poids de la composition totale, le rapport du polyacrylate à l'adhésif caoutchoutique étant de 2:98 à 96:4 ;
(c) une quantité à effet thérapeutique d'un médicament ou d'un mélange d'au moins deux médicaments pour administration transdermique de médicament, le médicament étant présent en une quantité de 0,1 % à 50 % de la composition totale ; et
(d) une polyvinylpyrrolidone soluble, la polyvinylpyrrolidone soluble étant présente en une quantité de 1 % à 20 % de la composition totale,
dans laquelle le rapport du médicament à la polyvinylpyrrolidone soluble est de 1:10 à 10:1, et
dans laquelle la quantité de polyvinylpyrrolidone soluble est suffisante pour solubiliser la totalité du médicament qui est présent en une quantité qui dépasserait sa solubilité dans une composition contenant un adhésif caoutchoutique et un polyacrylate, mais ne contenant pas de polyvinylpyrrolidone soluble.

2. Une composition de la revendication 1, comprenant 9 % à 94 % de polysiloxane à titre du composant adhésif caoutchoutique, 2 % à 85 % de polyacrylate, 1 % à 15 % de polyvinylpyrrolidone, 0 à 20 % de co-solvant, 0 % à 15 % d'activateur et 0,3 % à 30 % de médicament, en pourcentages en poids de la composition totale.

3. Une composition selon la revendication 1 ou 2, dans laquelle la polyvinylpyrrolidone a un poids moléculaire de 7000 à 54 000.

4. Une composition selon l'une quelconque des revendications 1 à 3, dans laquelle le médicament est choisi dans le groupe formé par les stéroïdes, les agonistes et inhibiteurs β₂-adrénergiques, les agents cardio-actifs, les agonistes cholinergiques, les tranquillisants, les anesthésiques, les analgésiques, les antinéoplasiques, les médicaments agissant sur le système nerveux central, les vasodilatateurs, les médicaments antiparkinsoniens, les médicaments antipsychotiques et les médicaments anti-inflammatoires non stéroïdiens.

5. Une composition selon la revendication 4, comprenant au moins deux médicaments.

6. Une composition selon la revendication 4, dans laquelle le médicament est choisi dans le groupe formé par les hormones oestrogènes conjuguées, les oestrogènes estérifiés, l'oestropipate, le 17β-oestradiol, l'équiline, le mestranol, l'oestrone, l'oestriol, l'éthinyl-oestradiol, le diéthylstilbestrol, la progestérone, la 19-norprogestérone, la noréthindrone, l'acétate de noréthindrone, le mélengestrol, la chlormadinone, l'éthistérone, l'acétate de médroxyprogestérone, le caproate d'hydroxyprogestérone, le diacétate d'éthynodiol, le noréthynodrel, la 17α-hydroxyprogestérone, la dydrogestérone, la diméthistérone, l'éthinyloestrénol, le norgestrel, la démégestone, la promégestone, l'acétate de mégestrol, le métaprotérénol, la terbutaline, l'albutérol, le carbutérol, le rimitérol, le fénotérol, le sotérénol, la nitroglycérine, le dinitrate d'isosorbide, le mononitate d'isosorbide, le sulfate de quinidine, le procaïnamide, le bendrofluméthiazide, le benzthiazide, le chlorothiazide, la nifédipine, la nicardipine, le vérapamil, le diltiazem, le timolol, le propranolol, le captopril, la clonidine, la prazosine, la choline, l'acétylcholine, la méthacholine, le carbachol, le béthanechol, la pilocarpine, la muscarine, l'arécoline l'alprazolam, le chlordiazépoxyde, le clorazépate, l'halazépam, l'oxazépam, le prazépam, le clonazépam, le flurazépam, le triazolam, le lorazépam, le diazépam, le thiopropazate, la chlorpromazine, la triflupromazine, la mésoridazine, la pipéracétazine, la thioridazine, l'acétophénazine, la fluphénazine, la perphénazine, la trifluopérazine, le chlorprathixène, le thiothixène, l'halopéridol, le brompéridol, la loxapine, la molindone, la lidocaine, la tétracaine, la dyclonine, la dibucaïne, la procaïne, la mépivacaïne, la bupivacaïne, l'étidocaïne, la prilocaïne, la benzocaïne, le fentanyl, la buprénorphine, la codéine, la nicotine, la papavérine, le butorphanol, l'hydromorphone, l'oxymorphone, la mécamylamine, le diclofénac, le fénoprofène, le flurbiprofène, l'ibuprofène, le kétoprofène, la naproxène, le piroxicam, l'acide δ-aminolévulinique, le déprényl, la sélégiline, la buspirone et le lisuride.

7. Une composition selon la revendication 3, comprenant 5 à 10 % de polyvinylpyrrolidone.

8. Une composition selon la revendication 5, comprenant un mélange d'un agent progestatif et d'un oestrogène.

9. La composition de la revendication 8, comprenant 71,3 % de polysiloxane, 5 % de polyacrylate, 10 % de polyvinylpyrrolidone, 3 % d'acétate de noréthindrone et 0,7 % d'oestradiol.

10. La composition de la revendication 6, comprenant 71,2 % de polysiloxane, 5 % de polyacrylate, 10 % de polyvinylpyrrolidone et 3 % d'acétate de noréthindrone.

11. La composition de la revendication 6, comprenant 26,2 % de polysiloxane, 45,3 % de polyacrylate, 9,5 % de polyvinylpyrrolidone, 7 % d'alprazolam et 10 % d'activateur.

12. La composition de la revendication 6, comprenant 12 % de polysiloxane, 60 % de polyacrylate, 5 % de polyvinylpyrrolidone et 10 % d'albutérol.

13. La composition de la revendication 6, comprenant 5 % de polysiloxane, 70 % de polyacrylate, environ 5 % de polyvinylpyrrolidone et 10 % d'acide δ-aminolévulinique.

14. La composition de la revendication 6, comprenant 52 % de polysiloxane, 20 % de polyacrylate, 10 % de polyvinylpyrrolidone et 5 % de fentanyl.

15. La composition de la revendication 6, comprenant 65 % de polysiloxane, 15 % de polyacrylate, 5 % de polyvinylpyrrolidone et 15 % de nicotine.

16. La composition de la revendication 6, comprenant 65 % de polysiloxane, 15 % de polyacrylate, 5 % de polyvinylpyrrolidone et 15 % de sélégiline.

17. La composition de la revendication 6, comprenant 5 % de polysiloxane, 60 % de polyacrylate, 5 % de polyvinylpyrrolidone et 30 % de kétoprofène.

18. La composition de la revendication 6, dans laquelle le médicament est le 17β-oestradiol.

19. Une composition selon la revendication 1, dans laquelle la composition a une température de transition vitreuse d'environ -70°C à 0°C.

20. Une composition selon la revendication 1, comprenant de plus un activateur.

21. Une composition selon la revendication 1, comprenant de plus une argile.

22. Une composition selon la revendication 21, dans laquelle l'argile est la bentonite.

23. Une composition selon la revendication 1, dans laquelle le médicament est choisi dans le groupe formé par la fluoxétine, la sertaline et la paroxétine.

24. Une composition selon la revendication 1, dans laquelle le médicament contient de la clonidine.

25. Une composition selon la revendication 1, dans laquelle le médicament est choisi dans le groupe formé par la bromocriptine, le lisuride et le pergolide.

26. Une composition selon la revendication 1, dans laquelle le médicament est choisi dans le groupe formé par le norgestimate, le norgestrel, l'acétate d'éthynodiol et le désogestrel.

27. Une composition selon la revendication 1, dans laquelle le médicament est choisi dans le groupe formé par l'ipsapirone et la buspirone.

28. Une composition selon la revendication 1, dans laquelle le médicament comprend de l'alprazolam.

29. Une composition selon la revendication 1, dans laquelle le médicament comprend du clonazépam.

30. Une composition selon la revendication 1, dans laquelle le médicament comprend de la clonidine.

31. La composition de la revendication 1, dans laquelle le médicament comprend de l'acétate de fludrocortisone.

32. La composition de la revendication 1, dans laquelle le médicament comprend du kétoprofène.

33. La composition de la revendication 1, dans laquelle le médicament comprend de l'oestradiol.

34. Un système d'administration transdermique de médicament comprenant une feuille de forme géométrique définie comprenant une composition telle que revendiquée dans l'une quelconque des revendications précédentes.

35. Un système selon la revendication 34, qui est sous la forme d'une unité d'administration individuelle.

36. Un système selon la revendication 34, qui comprend de plus un matériau de support superposé sur une surface de la composition, le matériau de support étant sensiblement imperméable au médicament, et une feuille détachable superposée sur la surface de la composition qui est opposée au matériau de support.

37. Un procédé de préparation d'une composition adhésive sensible à la pression pour l'administration transdermique de médicament, qui comprend l'opération de mélange de :
(a) un adhésif caoutchoutique en une quantité de 9 % à 94 % en poids de la composition totale ;
(b) un polyacrylate en une quantité de 2 % à 85 % en poids de la composition totale, le rapport du polyacrylate à l'adhésif caoutchoutique étant de 2:98 à 96:4 ;
(c) une quantité à effet thérapeutique d'un médicament ou d'un mélange d'au moins deux médicaments pour administration transdermique de médicament, le médicament étant présent en une quantité de 0,1 % à 50 % de la composition totale ; et
(d) une polyvinylpyrrolidone soluble, la polyvinylpyrrolidone soluble étant présente en une quantité de 1 % à 20 % de la composition totale,
dans lequel le rapport du médicament à la polyvinylpyrrolidone soluble est de 1:10 à 10:1, et
dans lequel la quantité de polyvinylpyrrolidone soluble est suffisante pour solubiliser la totalité du médicament qui est présent en une quantité qui dépasserait sa solubilité dans une composition contenant un adhésif caoutchoutique et un polyacrylate, mais ne contenant pas de polyvinylpyrrolidone soluble.

38. Une composition selon l'une quelconque des revendications 1 à 3, dans laquelle le médicament est choisi dans le groupe formé par l'énalapril, l'énalaprilat, la fluoxétine, l'acide niflumique, le piroxicam et la sertraline.
